(19)

Europäisches Patentamt
European Patent Office
Office européen des brevets

(11) EP 4 224 478 A1

(12) EUROPEAN PATENT APPLICATION

(43) Date of publication:
09.08.2023 Bulletin 2023/32

(51) International Patent Classification (IPC):
*G16B 25/10* [(2019.01)]

(21) Application number: 22155644.2

(22) Date of filing: 08.02.2022

(52) Cooperative Patent Classification (CPC):
G16B 25/10

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Universität zu Lübeck
23562 Lübeck (DE)

(72) Inventors:
• Dreßler, Franz
23564 Lübeck (DE)
• Perner, Sven
23562 Lübeck (DE)

(74) Representative: Hofmann, Matthias
Boehmert & Boehmert
Anwaltspartnerschaft mbB
Pettenkoferstraße 22
80336 München (DE)

Remarks:
The references to the drawing(s) no. 6K are deemed
to be deleted (Rule 56(4) EPC).

(54) METHOD AND SYSTEM FOR DETERMINING DIFFERENTIAL EXPRESSION

(57) The present invention provides a method for determining differential expression of a test analyte, the method comprising: obtaining transcription and/or protein measurements corresponding to a plurality of analytes, including the test analyte; determining a variation of the test analyte across the measurements of the test analyte and/or a correlation of the measurements of the test analyte with measurements of other analytes from the plurality of analytes, and determining a differentially expressed, DE, or non-differentially-expressed, nDE, likelihood of the test analyte based on the variation and/or the correlation.

Figure 1

EP 4 224 478 A1

**Description**

TECHNICAL FIELD

The present invention relates to a method for determining differential expression of a test analyte and an apparatus for determining differential expression of a test analyte. The present invention also relates to a computer-readable storage medium storing program code, the program code comprising instructions for carrying out such a method.

BACKGROUND

Accurate quantitation at omics scale is essential in many areas of biomedical research. With the advent of modern high-throughput quantitation methods such as RNAseq various normalization algorithms have been developed to correct for the variation in sample loading and quantitation. Most work so far has centred on RNA transcripts, for which packages like limma [1], NormalyzerDE [2] or BestKeeper [3] or specific algorithms such as variance normalization stabilization (VSN) [4, 5], median, LOESS and quantile normalization [6] have been developed.

As most actionable targets as well as effector molecules are proteins, and with clinical key questions unanswered by RNA and DNA analysis, the human proteome becomes increasingly relevant. While most normalization algorithms have linearly been expanded from nucleic acid to protein quantitation [7, 8], some algorithms such as DEqMS [9] or MAP [10] have been proposed to tackle proteome-specific problems. Still, a basic assumption remains at the heart of the most widely used algorithms: The majority of proteins or transcripts is not differentially expressed (nDE) [11].

The implications of this assumption have been discussed for RNAseq data in detail [12,13] and specific subset normalization methods have been proposed [14]. These methods, however, are based on either spike-in controls or the *a priori* definition of sample conditions. The latter is intrinsically problematic for scenarios in which stratification and clustering are sought based on unknown patterns of both proteins and samples - vital to expand prognostic and predictive information beyond the currently known pathological classifications and clinical stages.

While the assumption of the majority of proteins being nDE is a limitation in transcriptome normalization already [12], a relevant difference exists between the amplifying quantitation of RNA transcripts and the abundance-sensitive protein identification and quantitation by standard bottom-up liquid chromatography-coupled mass spectrometry (LCMS). Practically, this leads to a difference in quantitation depths, mirrored by the considerable differences in the typical number of identified transcripts or proteins compared to the theoretical spectrum. For proteomic analyses, this implies a bias towards high-abundance proteins [15, 16] and - in the setting of biological variation - towards (positively) DE proteins. Second, the actual function of proteins leads to considerable variation with little overlap in tissues of different biological states, most notably in cancer versus healthy parenchymatous tissue. Normalization to detect tumor-specific alterations, which are highly relevant for diagnostics and targeted therapies, thus needs to consider these prerequisites.

On the other hand, the increasing proteome coverage in proteomic analyses causes issues of multiple testing [17], and the increased size of the data input can impede clustering analyses [18]. To address these challenges, filtering of the data before downstream analyses has been proposed, but this again includes further assumptions and orthogonality requirements [18-20].

The use of internal nDE 'housekeeping' controls offers conservative and relatively unbiased normalization with fewer and less general assumptions about the underlying data structure. nDE controls have widely been used in quantitative real-time PCR as well as microarray analyses [3, 21-23] but the *a priori* selection of these proteins or genes remains difficult [3].

Thus, there is a need for an improved way of estimating the likelihood of a protein being DE or nDE.

SUMMARY OF THE INVENTION

The objective of the present invention is to provide a method for determining differential expression of a test analyte and an apparatus for determining differential expression of a test analyte, wherein the apparatus is configured to carry out the method of one of the previous claims, which overcome one or more of the above-mentioned problems of the prior art.

A first aspect of the invention provides a method for determining differential expression of a test analyte, the method comprising:

- obtaining quantitative measurements corresponding to a plurality of analytes, including the test analyte,
- determining a variation of the test analyte across the measurements of the test analyte and/or a correlation of the measurements of the test analyte with measurements of other analytes from the plurality of analytes, and
- determining a differentially expressed, DE, or non-differentially-expressed, nDE, likelihood of the test analyte based on the variation and/or the correlation.

Embodiments of the method of the first aspect present a conservative approach to estimate the likelihood of a protein being DE or nDE. This can be used to normalize proteomic datasets without *a priori* definition of neither experimental conditions nor internal spike-in or external nDE controls.

Determining a variation may be performed by determining a coefficient of variation. Determining a correlation may be performed by determining a correlation coefficient.

Determining a DE or non-DE likelihood may comprise assigning a DE status or a non-DE status to the analyte.

The method of the first aspect can be implemented on a computer. In particular, all steps can be carried out by a computer. The method may comprise an initial step of performing the quantitative measurements on the analytes. Preferably, the measurement instruments are controlled by the computer.

Experiments have shown that the method of the first aspect is applicable for a broad range of different analytes.

In a first implementation of the first aspect, the analyte is a gene, a metabolite, a lipid, an ion channel, pathogens, cells or cell types. For example, the quantitative measurement of an ion channel can be a measurement of a current through the ion channel.

In other example, the quantitative measurement can be obtained from an image of the analyte. For example, the quantitative measurement can be number of pathogens, cells or cell types and this can be obtained from image analysis, e.g. using automated image analysis such as segmentation of the images.

Generally, a preferred embodiment of the first aspect can be applied to anything that can be expressed in the form of a matrix of [analytes x samples]. Best results are achieved if a sample comprises a related measurement of several analytes, that the measurement variation is to be corrected over several samples and that there are no confounding measurement errors of certain analytes (these are then not to be separated from biological variation).

The variation of the analyte may be a coefficient of variation.

In an implementation, the coefficient of variation is determined as

$$\frac{\sqrt{Var(g_i)}}{\bar{g}_i}$$

wherein $Var(g_i)$ is a variation of measurements of the test analyte and $\bar{g}_i$ is a mean of an abundance $g_i$ of measurements of the test analyte.

Experiments have shown that using the coefficient of variation as defined above yields particularly good results.

Other choices of determining the variation that have yielded good results including determining the variation based on an interquartile range, e.g. using interquartile range as the variation.

In a further implementation, the method comprises a step of correcting a value of the variance of the test analyte based on a number of peptides used for quantification and/or based on a number of read variants.

The correlation may be a correlation coefficient.

In a further implementation, the correlation coefficient is a mean correlation coefficient, in particular the mean correlation coefficient $\bar{\rho}(g_i)$ that is determined by:

$$\bar{\rho}(g_i) = \frac{1}{N-1} \sum\nolimits_{n=1}^{N\backslash i} \rho(g_i, g_n)$$

wherein $g_i$ is the test analyte, $N$ is a number of the plurality of analytes, $\rho$ is a correlation coefficient, preferably a Spearman correlation coefficient.

Instead of a mean correlation value, a median correlation value or the mode of the correlation can be used.

In other embodiments, the correlation can also be determined using other measures such as Kendall's tau or Pearson's R.

In a further implementation, determining the DE or nDE likelihood of the test analyte comprises ranking the plurality of analytes based on the coefficient of variation and/or the correlation coefficient.

The ranking can be based only on the coefficient of variation or only on the correlation coefficient. However, preferably both are considered, e.g., a weighted sum of both. The weighting can be predetermined. Preferably, a weight for the coefficient of variation has a different sign than the weighting for the correlation coefficient.

In embodiments, the algorithm may also comprise sorting and ranking according to the variance, sorting and ranking according to a (e.g.: mean) correlation and then sorting according to a sum of the two ranks, wherein the sum may be a weighted sum.

In a further implementation, the ranking $R_i$ of the test analyte is determined as a sum of a first ranking function that assigns a higher ranking to a higher coefficient of variation and a second ranking function that assigns a higher ranking to a lower mean correlation coefficient.

In a further implementation, the ranking of the analytes by DE likelihood is used to filter the data for analytes with high DE likelihood in order to improve sensitivity and performance of downstream statistical analyses, such as unsupervised clustering, principal component analysis or statistical testing for differential expression.

In a further implementation, the method is a normalization method that comprises a further step of normalizing measurements based on one or more analytes with nDE status.

In a further implementation, the method comprises a further step of scaling down an array of correlation values and determining analytes with protein status based on a subset corresponding to the scaled-down array.

For example, calculating the mean correlation can be performed for each protein/transcript/analyte separately. This results in all pairs being calculated twice. The idea is to instead fill in a symmetric correlation matrix and then calculate the mean of each row (which is then the mean correlation for the corresponding protein/transcript). Practically, this can lead to large memory requirements and may be slower than a "brute-force" variant.

In an alternative implementation, the required correlations can be reduced overall by simply comparing only a subset of randomly selected analytes, e.g. proteins/transcripts. Instead of mere random selection, another filter can be used, e.g. selecting only the most abundant analytes.

In a preferred implementation, the method comprises a further step of determining a subset of the plurality of analytes and the determining a correlation of the measurements of the test analyte with measurements of other analytes from the plurality of analytes comprises determining the correlation of the test analyte only with other analytes that are part of the subset.

Due to the exponential computational effort, no matter which approach is used, significant computational time can be saved.

A further aspect of the present invention relates to an apparatus for determining differential expression of a test analyte, wherein the apparatus is configured to carry out the method of the first aspect or one of the implementations of the first aspect.

In an implementation, the apparatus further comprises a mass spectrometer and wherein the apparatus is configured to configured to access spectral libraries via a network connection. This has the advantage that an integrated device can be provided that can automatically carry out all necessary steps.

A further aspect of the invention refers to a computer-readable storage medium storing program code, the program code comprising instructions that when executed by a processor carry out the method of the second aspect or one of the implementations of the second aspect.

BRIEF DESCRIPTION OF THE DRAWINGS

To illustrate the technical features of embodiments of the present invention more clearly, the accompanying drawings provided for describing the embodiments are introduced briefly in the following. The accompanying drawings in the following description are merely some embodiments of the present invention, modifications on these embodiments are possible without departing from the scope of the present invention as defined in the claims.

FIG. 1     shows four diagrams that illustrate the structure of causality-based separation,

FIG. 2     shows diagrams that illustrate results from simulation data,

FIG. 3     shows diagrams that illustrate results from a first spike-in datasets,

FIG. 4     shows diagrams that illustrate results from a second spike-in dataset,

FIG. 5     shows diagrams that illustrate results from a third spike-in dataset,

FIG. 6     shows diagrams that illustrate results from a complex biological dataset,

FIG. 7     shows a diagram that illustrates biological function of proteins as ordered by an embodiment of the present invention,

FIG. 8     shows biological function of proteins order by an embodiment of the present invention, and

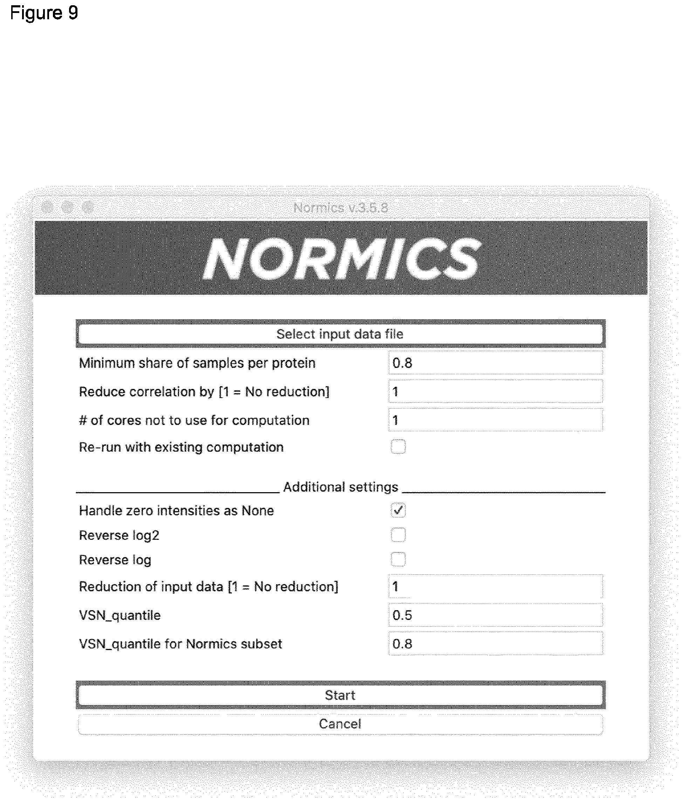

FIG. 9     is schematic illustration of a graphical user interface of the presented method.

DETAILED DESCRIPTION OF EMBODIMENTS

The foregoing descriptions are only implementation manners of the present invention, the scope of the present invention is not limited to this. Any variations or replacements can be easily made through person skilled in the art. Therefore, the protection scope of the present invention should be subject to the protection scope of the attached claims.

*Normics algorithm*

A preferred embodiment of the present invention is referred to as the "Normics algorithm".

The Normics algorithm uses the data-inherent correlation structure (ICS) as a feature of the input data and uses it together with the variance structure to order proteins by their likelihood of being nDE. We then perform normalization with established algorithms on these subsets only. The resulting parameters are extended to the entire dataset. The complete theoretical approach is described below. Briefly, the overall variance or scatter of each protein results from a combination of different sources of variation. Among these, variation due to unintentional loading or technical measurement differences affects all proteins of every sample. In contrast, the 'true' biological variation affects only DE proteins. The combined effect of these two main sources can be measured by the variance and corrected for intensitiy-dependent distortions by the coefficient of variation ($CV$). It is expected to be larger for DE proteins and has been used by Czechowski et al. to select nDE controls [23], conversely by Bourgon et al. to filter for DE transcripts [20] and in a more complex model-based approach by Calza et al. [24, 25]. The latter but also the $CV$ itself implies different assumptions about variation measures and quantitative relationships.

We therefore include another separation factor for the identification of nDE controls. Due to the causality between sample loading variation and increased variation of all proteins, the correlation between nDE and DE proteins can be expected to be positive. In contrast, biological variation can reasonably be expected to be both positive and negative, leading to both positive and negative correlations between DE proteins of different biological sets. As a result, the mean correlation ($\overline{\rho}$) of nDE proteins with all other proteins will tend to be higher compared to DE proteins, which correlate positively with nDE proteins but negatively with other DE proteins that are co-regulated but in opposite direction. Also, the size of the nDE set can be expected to be larger than any of the coregulated DE sets, further solidifying the approach.

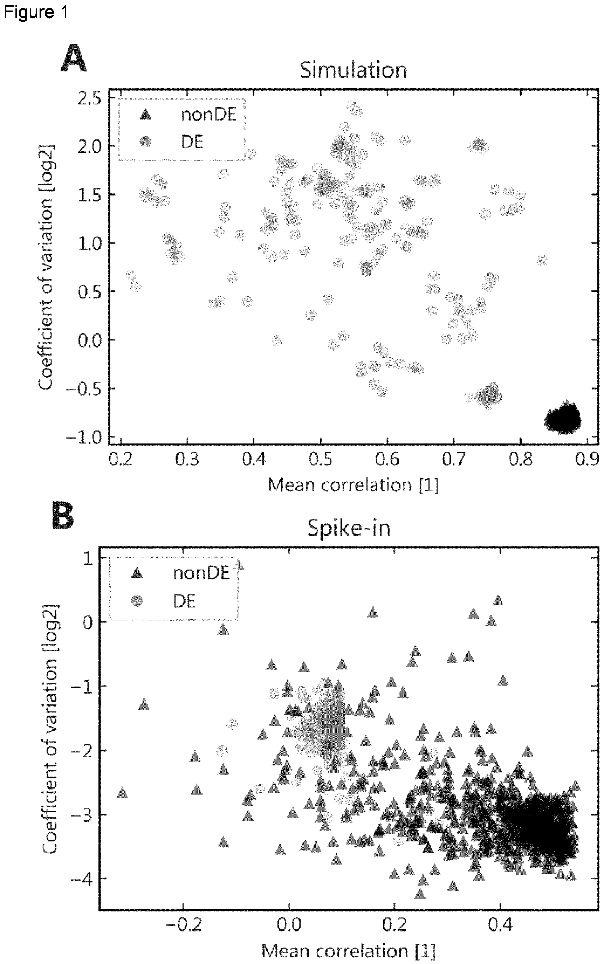

We order all proteins by these features (ascending for $CV$ and descending for $\overline{\rho}$) and calculate the rank sum R, which we interpret as the likelihood of each protein to be DE. An nDE subset is chosen for downstream normalization of the entire dataset based either on discernable cluster formation (Fig. 1B) or by *a priori* expectation of the share of DE proteins (similar to setting the VSN quantile but with a higher maximum share of DE proteins). Either standard median normalization [2] (Normics$_{median}$) or VSN [5] (Normics) is applied to the normalization subset. Median normalization was chosen as it does not alter the data structure (as opposed to quantile and LOESS). VSN in turn provided superior performance in a previous comparative study [7].

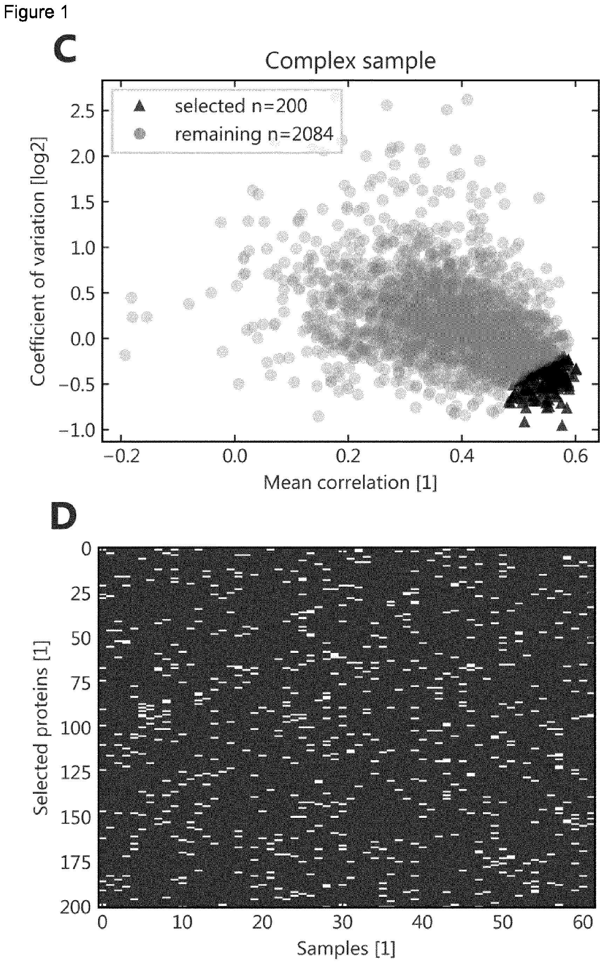

Protein candidates for subset normalization must be present in all samples (as with all normalization housekeepers/channels) or, in our case with multiple proteins in the normalization subset, at least in the vast majority of samples. The respective thresholds are set in the Normics graphical user interface (GUI), and a visualization of the missing values in the normalization subset is shown (Fig. 1D). Independent of missingness, all proteins of a dataset are normalized with the Normics subset.

*Normics implementation*

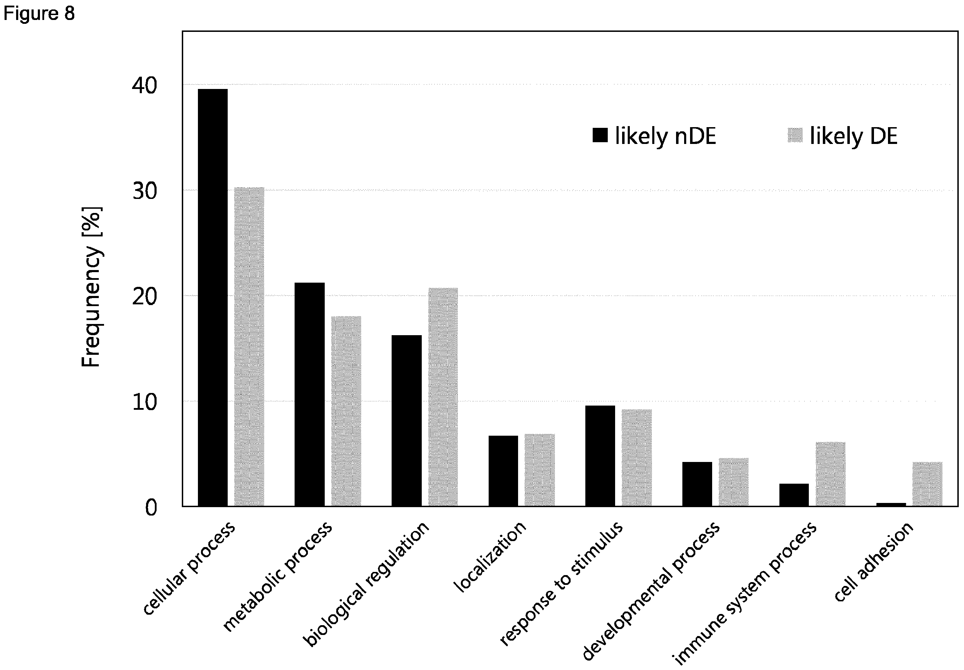

The algorithm was implemented in Python 2.7.17 using the packages numpy 1.16.1, scipy 1.2.2, matplotlib 2.2.4, seaborn 0.9.1, pandas 0.24.2 and multiprocessing 2.6.2.1. Due to the time-intensive computation of order $0[0.5 (i^2 - i)]$ the pairwise calculation with subsequent insertion into a symmetric correlation matrix [$i, i$] was tried to achieve efficient computation. In practice, however, this requires considerably more memory space and proves to be slower than computing protein correlations entirely one by one. Additionally, larger arrays can be scaled down and nDE proteins identified from these subsets only (implemented as option in the algorithm). Calculations were parallelized using the multiprocessing package. The GUI of the Python implementation is illustrated in Fig. 8.

*Theoretical Approach*

Given a matrix with rows of $i$ proteins with intensities $g$ and $j$ columns of samples the overall variation or scatter across sample values of each protein results from a combination of different sources. Among these, variation due to unintentional, sample-specific loading or technical measurement differences affects all proteins of each sample ($Var_{all}$). In contrast, the 'true' variation affects only DE proteins ($Var_{DE}$) and can be considered as biological, sample-specific variation. Last, the intensity quantitation itself comes with a random error $\varepsilon$, which is generally considerably smaller than $Var_{all}$ and $Var_{DE}$ [38]. These main sources of variation can be quantified as variance and expected to follow:

$$(1)\ Var(g_i) = Var_{all} + Var_{DE,i} + \varepsilon\ for\ g_i \in DE$$

$$(2)\ Var(g_k) = Var_{all} + \varepsilon\ for\ g_k \in nDE$$

$$(3)\ Var(g_i) > Var(g_k)\ for\ g_i \in DE\ and\ g_k \in nDE$$

However, the signal intensity levels *g* (i.e. abundances) of different proteins are unequal, with higher protein abundance being correlated with higher variance. While the relationship between protein abundance and variance is not always strictly linear (as considered by VSN), the coefficient of variation (CV) removes most of the bias:

$$(4)\ \frac{\sqrt{Var(g_i)}}{\bar{g}_i} > \frac{\sqrt{Var(g_k)}}{\bar{g}_k} for\ g_i \in DE\ and\ g_k \in nDE$$

This approach has been used by Czechowski et al. to select nDE controls [23] and in a more complex model-based approach by Calza et al. [24, 25], implying further assumptions about the data structure and share of nDE proteins/transcripts. Apart from the non-linearity demonstrated by Huber et al. [4] the validity of this relationship also depends on a) the adequacy of the variance as a means of variation and b) the extent and distribution of variation compared to the level of $\bar{g}_i$ (or the modelled effects) as pronounced variation can lead to under- or over-correction (by eq. 4 or a modelling approach).

It is therefore sensible to include another separation factor for the identification of nDE controls. The foundation of equations 1 to 3 is formed by the connection between sample loading variation and increased variation of all proteins. Due to this logical link, the correlation between nDE and DE proteins can be expected to be positive. In contrast, biological variation can reasonably be expected to be both positive and negative, leading to both positive and negative correlations between DE proteins of different biological sets. As a result, the mean correlation of nDE proteins with all other proteins will tend to be higher compared to DE proteins, which correlate positively with nDE proteins but negatively with other DE proteins that are co-regulated but in opposite direction.

$$(5)\ -1 \leq \rho(g_i, g_l) \leq 1\ for\ g_i, g_l \in DE$$

$$(6)\ 0 \leq \rho(g_k, g_i) \leq 1\ for\ g_k \in nDE\ and\ g_i \in DE$$

$$(7)\ 0 \leq \rho(g_k, g_h) \leq 1\ for\ g_k, g_h \in nDE$$

The resulting mean correlation coefficient

$$(8)\ \bar{\rho}(g_i) = \frac{1}{N-1} \sum_{n=1}^{N \setminus i} \rho(g_i, g_n)$$

can thus be expected to be larger in nDE than DE proteins.

$$(9)\ \bar{\rho}(g_i) < \bar{\rho}(g_k)\ for\ g_i \in DE\ and\ g_k \in nDE$$

This can be expected to hold true even for datasets that do not contain relevant inverse regulation as long as the size *N* of the nDE subset exceeds the size of the largest coregulated DE subset (which is a less rigorous assumption than assuming the majority of all proteins to be nDE).

$$(10) \qquad \bar{\rho}(g_i) < \bar{\rho}(g_k) \; for \; g_i \in DE_m \; and \; g_k \in nDE \; and \; \max\left[N(DE_m)\right] < N(nDE)$$

While the Spearman correlation coefficient $\rho$ is used as a robust (yet coarse with low sample sizes) correlation measure, the interplay of overall variance, DE variance and set sizes of co-regulated DE as well as nDE proteins will influence the resulting differences. Most importantly, due to the abundance bias of LCMS quantitation, the suppression of weaker signals by more abundant peptides can be observed in spike-in datasets, which can lead to a pseudo-inverse correlation of DE and nDE proteins. Complex biological samples, however, can be expected (as outlined above) to include nDE proteins of various expression levels as well as multiple sets of both up- and downregulated DE proteins, which renders biased signal suppression unlikely. Also, systematic differences in sample loading between biological conditions could create pseudo-inverse correlations but are avoided by adjustment to protein or peptide concentration before the measurement. Last, the relative levels of $Var_{all}$ and $Var_{DE}$ affect the resulting correlation structure. It can reasonably be assumed that $Var_{all}$ is quantitatively relevant compared to $Var_{DE}$ as otherwise normalization would not be necessary [11] (an assumption that is implicitly made by all normalization algorithms [39]). Even if this was not true, ordering by $CV$ (equation 4) as well as the inverse DE coregulations would (partially) prevent the algorithm from reducing biological variance by mistake.

Taken altogether, both approaches provide robust *a priori* reasoning to rank proteins by their likelihood of being nDE - descending for $\bar{\rho}$, ascending for $CV$. We combine both rank indices $I$ into a rank sum:

$$(11) R_i = I_\uparrow[CV(g_i)] + I_\downarrow[\bar{\rho}(g_i)]$$

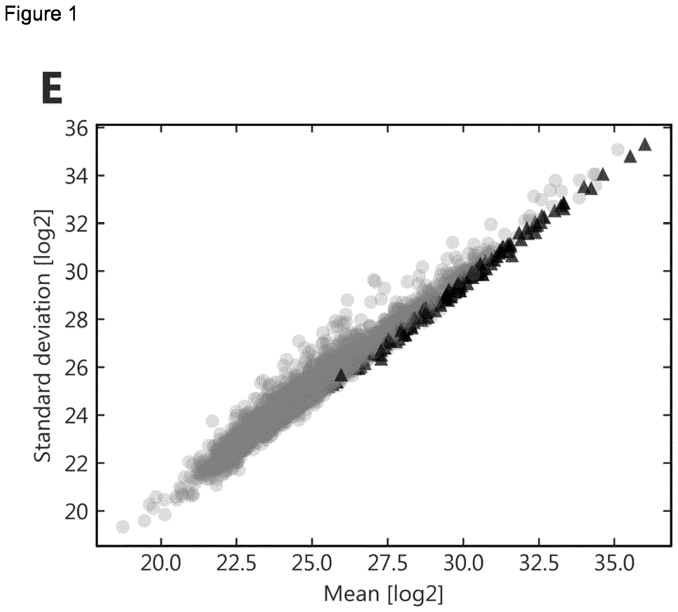

Based on this ranking a normalization subset is chosen for downstream normalization of the entire dataset. This choice is based either on discernable cluster formation (Fig. 1B) or by *a priori* expectation of the share of DE proteins (similar to setting the VSN quantile but with a higher maximum share of DE proteins). If no reasonable expectation about the data can be made, a conservative choice of 10 % nDE proteins can be used. Either standard median normalization [2] (Normics_{median}) or VSN [5] (Normics) is applied to the normalization subset. The sample-wise normalization factors or factors and offsets (VSN) are retrieved and applied to the complete dataset. In case VSN is used, the data is transformed to the generalized logarithm as defined by Huber et al. [5] and log2 transformed otherwise. In both modes, setting the quantile for the least trimmed squares (LTS) estimator [40] in VSN to 0.8 and the median itself further strengthen the approach against outliers. If VSN is chosen, sufficient sample size of the subset of at least n=50 has to be ensured [4]. Furthermore, sufficient coverage of the range of protein intensities is important (Fig. 1E).

*Application to further* omics *levels*

Currently, proteomic analysis is only a small subset of omics analyses. These include all quantitative measurements of molecules on a large scale, i.e., hundreds and thousands of analytes per measurement. Examples include quantification of RNA molecules (e.g. by RNA sequencing or microarrays), determination of copy number variations in DNA sequencing or quantification of smaller molecules such as metabolites, drugs, (environmental) substances or toxins. The presented approaches can be used in all these scenarios for normalization of different samples. Best results are achieved when there is mainly a technical/non-biological variance that affects the analytes of a sample equally. For cases where this is not given, the approach can still be used if the analytes can be divided into groups that sufficiently fulfill the conditions. As an example, the analysis for Copy Number Variations i.e. amplified genome segments can be given. The sequencing depth for quantification of the genome set present is not always homogeneously distributed across all possible sequences, but normalization of the measurement for specific strata of measured intensities (i.e. reads) is possible analogous to protein intensities.

*Other algorithms and implementations*

VSN was performed as implemented in the vsn2 function of the vsn package (vsn 3.58.0 using Biobase 2.50.0 and BiocGenerics 0.36.0 with lts.quantile=0.5 to allow for maximum robustness against DE proteins). Implementations for median, quantile and cyclic LOESS normalization were from the NormalyzerDE package (version 1.5.4) [2]. Additionally, MaxLFQ had been included in some of the datasets (see below).

*Generation of simulation data*

To test the postulated relationships we simulated complex proteomic datasets as - to our knowledge - real test datasets with complex patterns of up- and downregulation in multiple groups and known true positive DE proteins do not exist. It is this scenario with unknown groups that is highly relevant in screening approaches in oncological and pathological research.

Using the random module in Python we generated 250 datasets of 1000 proteins and 40 samples each. Intensities $g$ of proteins $i$ and samples $j$ were created by

$$g_{ij} = r_j \, L_i \, 2^{\,D_{ij} \, C_i(Q_{DE})} \, \mathcal{N}(1, \varepsilon)$$

with intensity level $L_i$ from $\mathcal{N}(10, 3)$, relative sample ratio $r_j$ from $[0,10]$ and correlation direction $C_i(Q_{DE})$ being -1 with probability $Q_{DE}$ and 1 otherwise. The differential expression matrix $D$ was created by attributing proteins to nDE ($D_{ij} = 0$) and DE groups by probability $p_{DE}$. If proteins were DE, a set of coregulated proteins was created by randomly deciding which samples were regulated with minimum 10 % and maximum 90 % of all samples being involved in this set (as otherwise the logical discernability from nDE proteins vanishes). The set size was iteratively determined by probability $p_{set}$. The differential expression factor for each sample within a set was drawn from $\mathcal{N}(\mu_{DE}, \sigma_{DE})$ with $\mu_{DE}$ from N(0, 1) and $\sigma_{DE}$ from $[0.1, 10.0]$. The level of $\varepsilon$ was chosen for an entire simulation dataset from $[0.00, 0.25]$.

*Evaluation of simulation data*

The normalized datasets were compared to the true dataset, i.e. the input data corrected by the known relative sample ratios and log2 transformed. To compare the data structure, both the true expression of a protein as well as the normalized data were z-score transformed and the squared differences (errors) summed up (SSE).

*Spike-in datasets*

To investigate the performance in real datasets with known true positives, we used publicly available spike-in datasets (Tbl. 1): A two-step E. coli spike-in with label-free quantitation (dS1) by Cox et al. (PXD000279; proteomecentral.proteomexchange.org) [26] similarly used for comparisons by Zhu et al. [9]; A three-step E. coli spike-in with TMT quantification (dS2) by Zhu et al. (see table 1 below) [9] and a protein standard (UPS1) spike-in with label-free quantitation (dS3) by Pursiheimo et al. (PXD002099; ebi.ac.uk/pride) [27], similarly used by Valikangas et al. [7].

**Table 1:** *Datasets.* FFPE = formalin-fixed, paraffin-embedded, TMT = Tandem mass tags

| Dataset | Type | Source | Fractionation | Quantification | Share of DE proteins |
|---|---|---|---|---|---|
| dS1 | E. Coli spike-in (two levels) | [26] | yes | Label-free | 29% |
| dS2 | E. Coli spike-in (three levels) | [9] | yes | TMT | 22% |
| dS3 | UPSi spike-in | [27] | no | Label-free | 3% |
| dC1 | Mouse fresh-frozen | [28] | no | Label-free | N/A |
| dC2 | Human tissue FFPE | [29] | yes | Label-free | N/A |

*Complex datasets*

To include more realistic data in our comparative analyses, we used published data that intrinsically provided a high probability of a high share of DE proteins (Tbl. 1): A mouse study by Vehmas et al. investigating the proteomic effects in the liver with knock down of a central metabolic enzyme (dCi; PXD002025; ebi.ac.uk/pride) [28] and a study in human tissue by Sohier et al., in which distinct pathological types of colorectal adenoma were compared on proteome level (dC2; PXD014511; proteomecentral.proteomexchange.org) [29].

*Evaluation of datasets*

Protein identification and intensity tables from the different datasets were used as input for the normalization algorithms. When label-free quantification had been performed (dSi, dC2) with the MaxQuant software (MaxLFQ) both datasets were used as input for further normalization as MaxLFQ combines peptides and fractions more accurately into protein abundances (at the cost of normalization by assuming minimal differential expression across the dataset) [26]. For all comparative analyses, only proteins with fewer than 20 % missing values across all samples (50 % in dC2) were included. For the calculation of differential expression, log2 or generalized-log (glog) transformed values were transformed back to avoid altering the original data structure. Statistical significance was tested for by two-sided Student's t-tests or Wilcoxon-Mann-Whitney-U for dC2 (in which the number of available replicates per group was sufficiently high to avoid quasi-discrete p-values), with missing values being excluded. P-values were FDR-corrected by the Benjamini-Hochberg method as implemented in the python statsmodels package (0.8.0) [30]. Figures were created in Python 2.7.17 using the packages numpy 1.16.1, scipy 1.2.2, matplotlib 2.2.4, seaborn 0.9.1, pandas 0.24.2. The Venn diagrams were created using the online tool InteractiVenn [31].

*Integration in quantitative Measurement Technology*

Our approach was based on the causality of the different sources of variance that arise during the measurement process of an analyte in e.g. a mass spectrometer with high-throughput liquid chromatography (LCMS). In principle, these measurement errors affect all portions of a complex analyte mixture, e.g., a protein extract consisting of several thousand different proteins of a tissue. This is in contrast to the biological variance across different samples from different patients. Separating this from the "technical" variance, i.e. variance caused by sample preparation and measurement, is the goal of normalization.

The example of protein or peptide quantification (proteins are broken down into their peptides) using LCMS illustrates the complexity of measurements at the medical level: due to the large number of proteins in the sample, sometimes with very different amounts, it is often necessary to divide the peptides of the sample into different fractions. This is done with biochemical separation methods that are as different as possible (orthogonal) to the separation by chromatography (the latter is immediately upstream of the analysis of the spectra with the mass spectrometer). These separations are imprecise and affect peptides differently, resulting in overlaps between fractions, i.e., one peptide is found in several fractions. At the same time, there are systemic measurement errors and biases between the measurements of the different fractions, and the total amount of peptides additionally varies. The measured peptide intensities from the different fractions must be combined back into one result after the measurements to determine the amount in the total sample.

The causality of the sources of variance can be divided here - analogous to the normalization across patients - into "true" variance due to the intended separation of the peptides and "unintended" variance due to the systematic measurement errors between the fractions. Since a very small fraction of peptides can be assumed to be consistently found in different fractions, our algorithm is able to reduce the variance between the different fractions of a sample already immediately after the identification of the peptides based on their measured spectra stepwise and without human interaction in an autonomous process. Likewise, the automatic correction of a data set from multiple samples is possible if the number of associated samples has been defined beforehand. The cut-off for the minimum fraction of non-varying (housekeeping) peptides and proteins can thereby already be defined in the measurement settings and thus a complete technical unit can be developed, consisting of mass spectrometer, associated data processing system with online-based query of the publicly available spectral libraries and self-sufficient stepwise normalization process on the associated data processing system using the sequence of mathematical steps we have developed. These are:
Filtering of eligible analytes based on their presence in different fractions/samples according to preset threshold.

- calculation of the coefficient of variation CV (as outlined above)
- calculation of all correlation coefficients between all analytes
- formation of the mean $\bar{\rho}$ per analyte
- sorting (ascending) according to the size of CV
- assignment of rank R(CV) per analyte
- sorting (descending) according to the size of $\bar{\rho}$
- assigning of rank R($\bar{\rho}$) per analyte
- forming of the sum of R(CV) and R($\bar{\rho}$)
- sorting according to this sum
- calculating of the sample/fraction-specific correction factors using the medians of the intensities of the subpopulation of analytes that are at the top positions in the list sorted according to the previous sorting step (percentage to be specified, e.g. choosing a threshold value between 5% and 30%, in particular between 8% and 15%).

As outlined above, the analyte can be for example a protein or a peptide.

**Results**

*Patterns of ICS and CV-based data separation*

Fig. 1 illustrates the structure of ICS and CV-based data separation. (A): Exemplary simulation dataset with the known differentially expressed (DE) and non-DE (nDE) populations; (B): In analogy to subplot A with a spike-in dataset (dS2); (C): Data structure for a complex sample (dC2) without known DE proteins, the top 200 proteins selected by the algorithm to be likely nDE are marked in blue; (D): Visual display of missing data points in the chosen normalization subset of C; (E): Relationship between protein signal intensity and variance to assess sufficient coverage of the data range by the nDE subset from subplot C (blue), relevant if VSN is chosen as downstream normalization method.

Fig. 1 demonstrates the distribution of proteins by the measures defined above: Simulation data exhibits clustering of nDE proteins around values of minimal $CV$ and maximum $\overline{\rho}$ as predicted (Fig. 1A). The same relationship can be found in spike-in data (Fig. 1B) with easily identifiable clusters of DE and nDE proteins, which can be expected due to the dichotomous character of the underlying artificial population. Similar to the simulated data, proteins in complex datasets form a cloud with a negative slope, yet a more scattered, blurred arrangement of data points (Fig. 1C). Missingness of the subset data is exemplary displayed in Fig. 1D, which is also shown to the user to ensure unbiased distributions of the normalization subset proteins across samples. Fig. 1E demonstrates the distribution in terms of variance and mean (sufficient coverage is relevant for choosing VSN as algorithm of downstream normalization).

*Performance with simulated data*

To cover a wide range of parameter combinations, key parameters were stochastically chosen across relevant ranges of technical and residual variance, unbalanced up- and downregulation, different sizes of coregulated sets and varying quantitative differences between DE and nDE proteins. Most importantly, the effect of high shares of DE proteins was modelled by parameter $p_{DE}$. 250 datasets of 1000 proteins and 40 samples each were created and normalized with the different algorithms (for Normics with the top 10 % used for normalization).

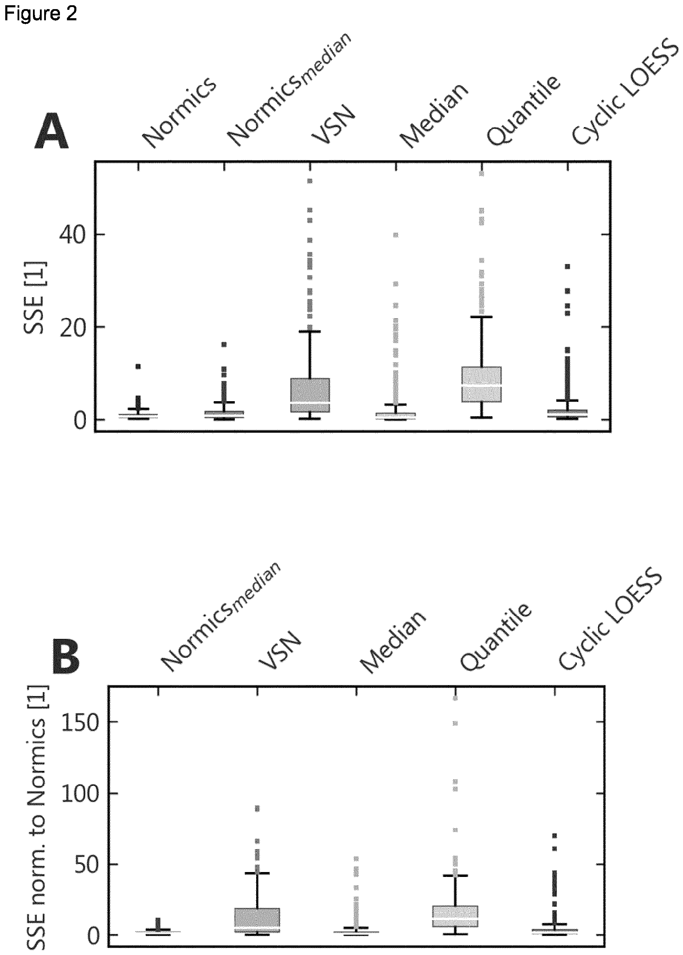

The subplots of Fig. 2A-B show boxplots of the sum of squared errors (SSE) distributions for the different algorithms; (B): In analogy to subplot A but with the SSE of each simulation run normed to the Normics result; (C)-(H): SSEs (y axis) of Normics and VSN across the stochastically varied range of the respective parameters (x axis); each dot represents a different simulation (N=250); dashed line in subplot D denotes the theoretical threshold (as explained in the text).

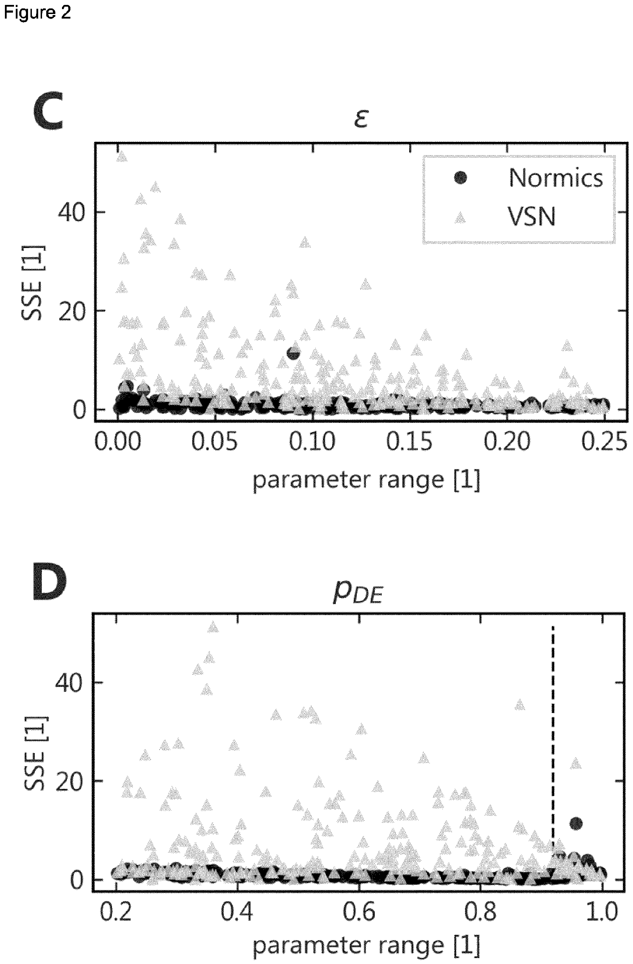

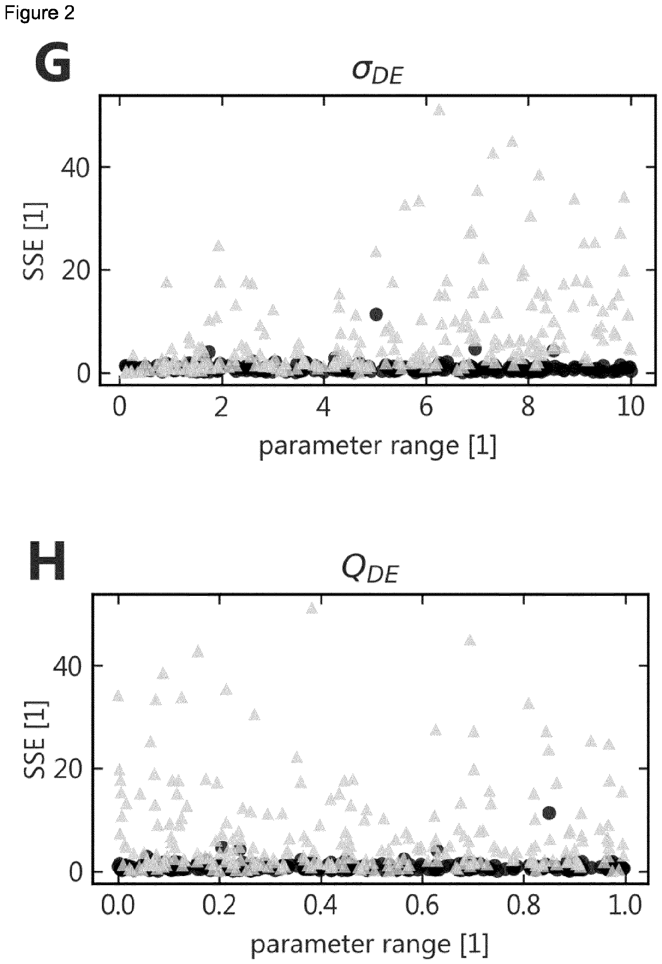

Fig. 2A-B visualizes the resulting distributions and shows considerably reduced errors in the data structure with Normics compared to the other normalization algorithms. While median normalization also performed well, it produced a considerable number of outliers. To take a closer look at the effects of subset-based normalization with Normics, Fig. 2C-H compares the results for Normics and VSN across the parameter ranges. Subplot D demonstrates the robustness of Normics against a high share of DE proteins up until $p_{DE}$=0.93, well in line with a theoretical value of >0.92 (dashed line; lts.quantile for VSN = 0.8, top 100 out of 1000 proteins leads to 0.08). Subplot G shows an increase in error with higher DE variation for VSN. Similarly, VSN errors get higher with the extent of unbalanced up- and downregulation (Fig. 2H). In both scenarios Normics performs robustly.

*Performance with* spike-in *datasets*

Simulation data builds upon assumptions implicitly made by the definition and structure of data creation, while datasets with known true positives offer realistic data and variance structure. We tested the performance of our algorithm with three different datasets comprising varying levels of spike-in DE proteins and different quantitation modes (label-free and TMT). To compare the performance of the different algorithms we refrained from evaluating descriptive parameters such as minimization of selected variances and took the position of an actual user applying either algorithm to normalize their data. Setting the significance level for FDR-corrected p-values to standard 0.05 we focused on the number of correctly identified DE and falsely selected nDE proteins as well as the quantitative structure of the normalized data.

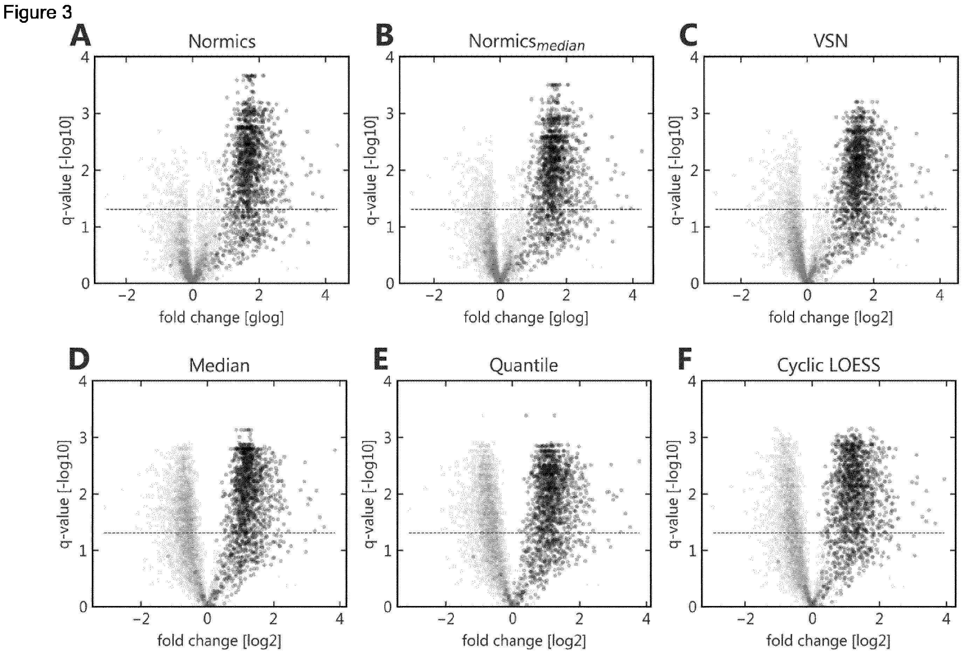

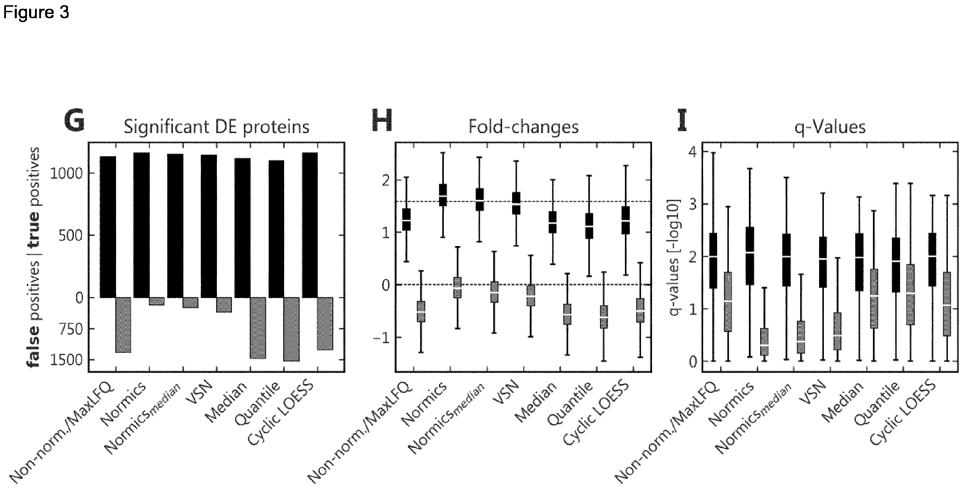

Fig. 3 shows results from spike-in dataset dS1. (A)-(F): Volcano plots of the data normalized with the respective algorithms, grey = non-DE, black = DE; (G): Number of proteins correctly (black) and falsely (grey) identified as DE by the respective algorithms; (H): Fold changes of the true and false positives, dashed lines indicate correct value; (I): Distribution of the q-values of true and false positives.

For dS1 (Fig. 3) we used the MaxLFQ-quantified data as input with similar results for the raw data (not shown). Both Normics approaches outperformed the other algorithms in terms of true positives and showed very few false positives (Fig. 3A-B, G) using 30 % of the downscaled input data (n=150). The correct quantitative difference was retrieved for the DE subset while the false positive nDE proteins were accurately centered around a log2 fold change of 0 (Fig. 3H). VSN alone showed comparable performance (yet with more false positives; Fig. 3C) but all other normalization algorithms including MaxLFQ demonstrated high numbers of false positives (in part exceeding the number of true positives) with considerable log2 fold changes centered around 0.5 (Fig. 3D-H). The quantitative differences of the DE proteins were less accurately retrieved (Fig. 3H).

We further investigated the quantitative behavior of our algorithm in datasets dS2 and dS3.

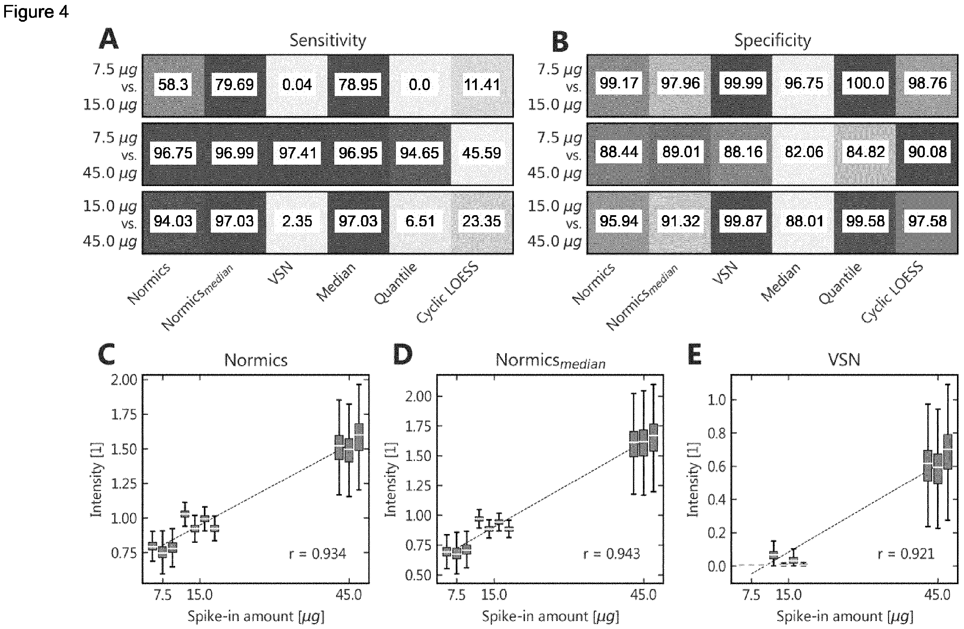

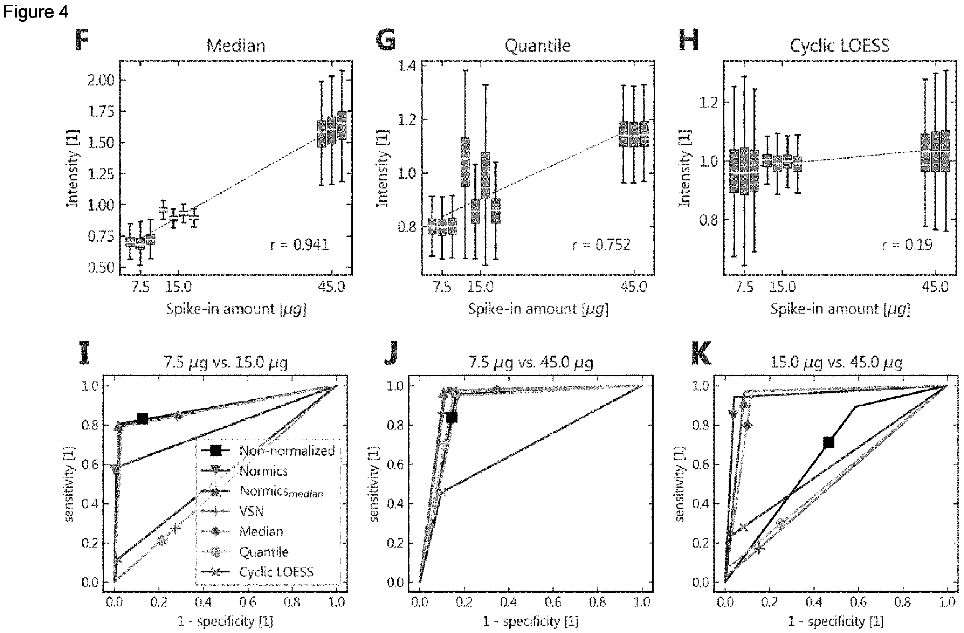

Fig. 4 shows results from spike-in dataset dS2. (A)-(B): Heatmaps (darker is higher) showing sensitivity and specificity of the DE proteins identified by the specific algorithm (greyscale range set individually per comparison); (C)-(H): Quantitative range of the normalized data; dashed line is the linear regression of the data pooled across replicates; boxplots show data distribution per replicate, grouped around their true x axis value; (I)-(K): Receiver operating characteristic of the overall performances.

In dS2 Normics and especially Normics$_{median}$ correctly identified most DE proteins with a sensitivity between 80 and 97 % while only few false positives were selected (specificity 89 - 98 %; Fig. 4A-B). n=250 (22 %) of a randomly downscaled protein subset were used. The quantitative differences were correctly retrieved (Fig. 4C-D). Only median normalization showed comparable performance but with reduced specificity (82 - 97 %).

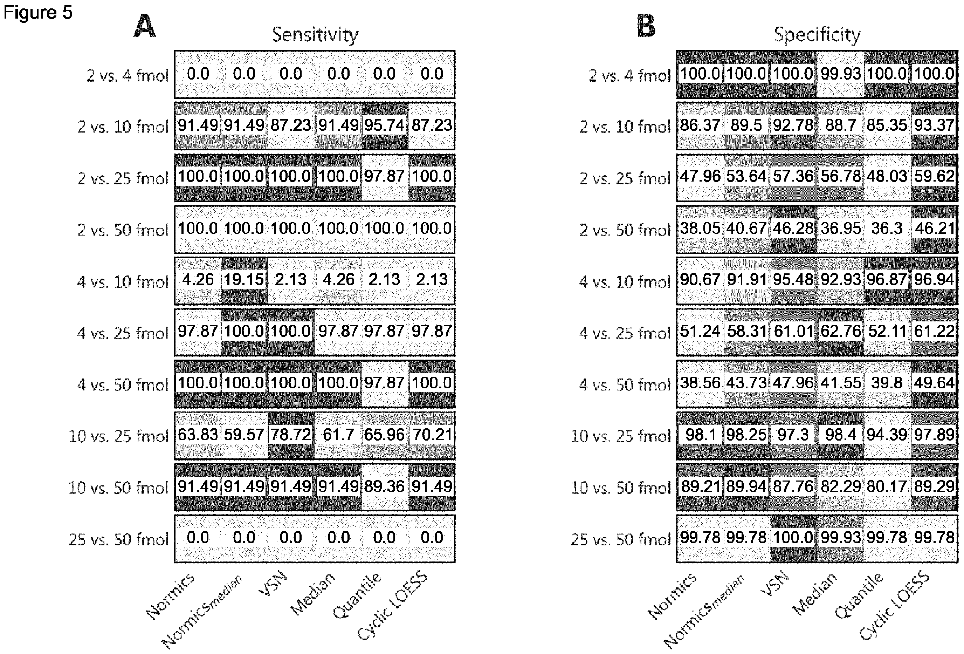

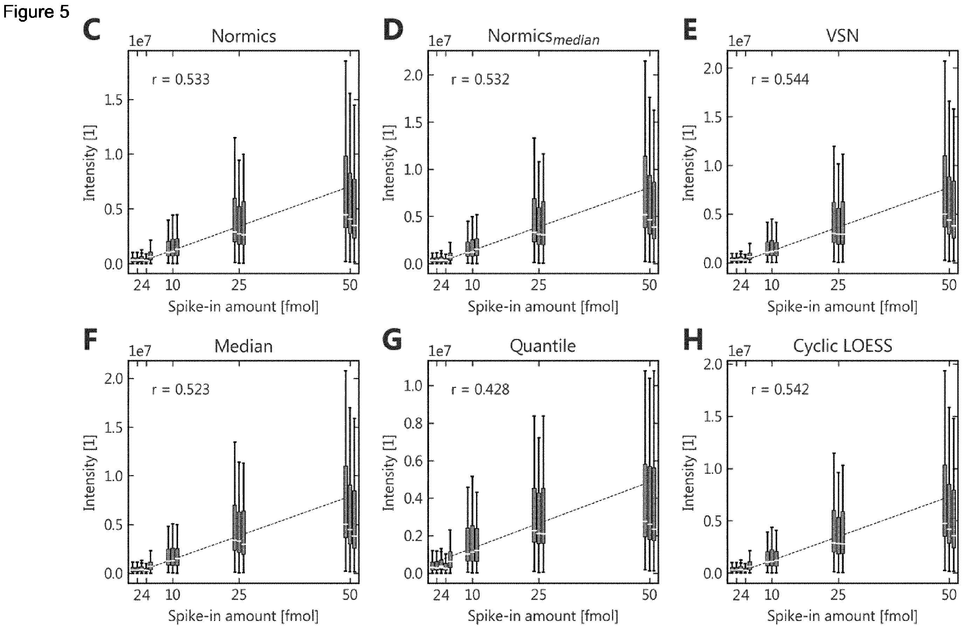

Fig. 5 shows results from spike-in dataset dS3. AA)-(B): Heatmaps showing sensitivity and specificity of the DE proteins identified by the specific algorithm (greyscale range set individually per comparison); (C)-(H): Quantitative range of the normalized data; dashed line is the linear regression of the data pooled across replicates; boxplots show data distribution per replicate, grouped around their true x axis value.

In dS3 (Fig. 5) five different levels of the protein standard UPS1 had been spiked into a yeast lysate. In the resulting comparisons Normics and especially Normics$_{median}$ again proved to identify DE proteins with high sensitivity and specificity - comparable to VSN (n=250; 18 % of all proteins were used). The quantitative range was similarly retrieved by all algorithms.

*Performance with complex datasets*

One of the main use cases for omics normalization is a screening approach with a dataset of multiple groups with complex (co-)regulation patterns. dC1 was chosen as the overexpression of a central metabolic and endocrine enzyme leads to a high probability of numerous DE proteins. Additionally, in this dataset cross-validation of DE pathways had been performed with transcriptome analysis. With the true positives unknown in dC2, we focused on comparing the p-value distribution as well as the number of identified DE proteins, as relevant pathological differences were expected.

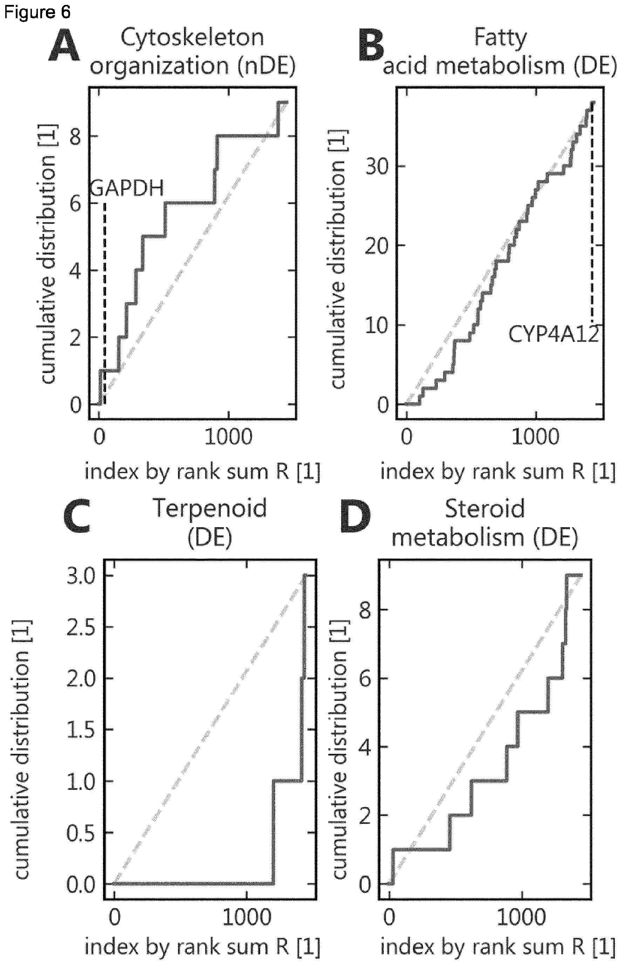

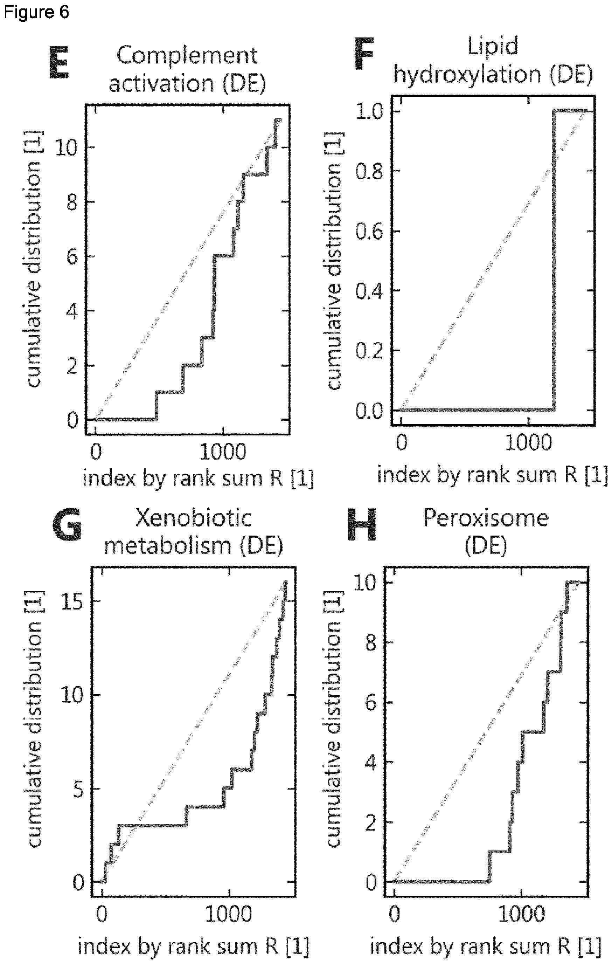

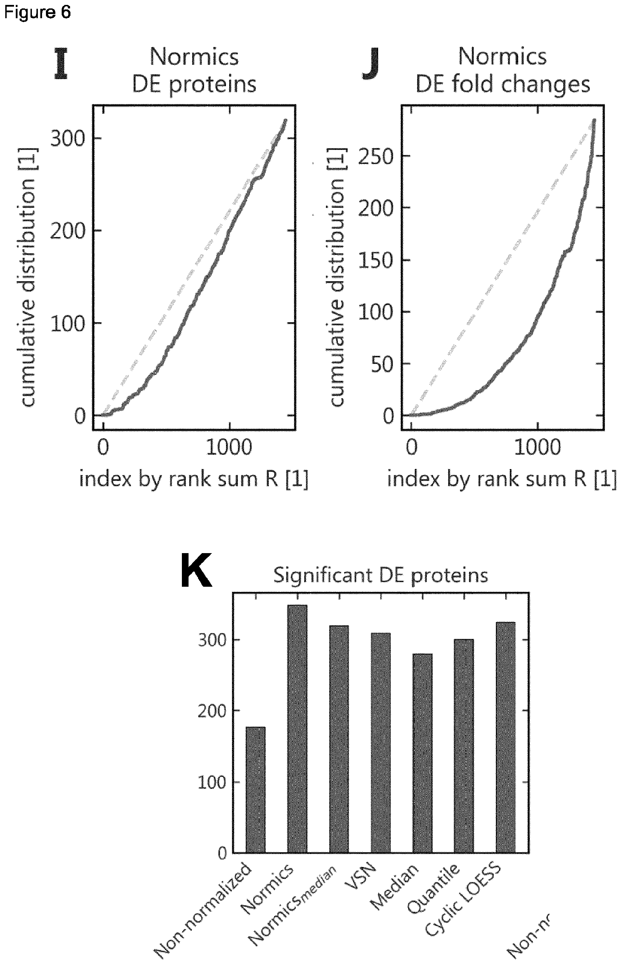

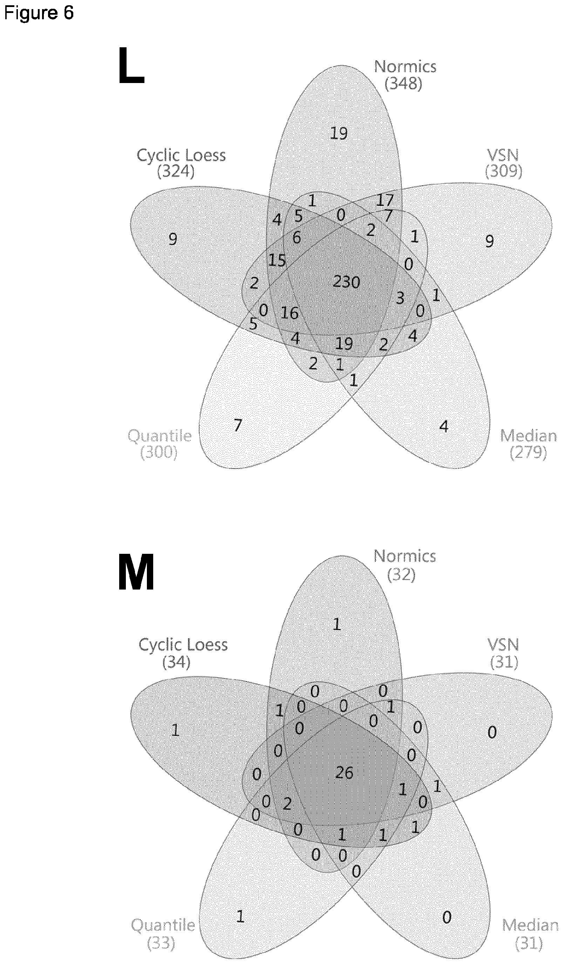

Fig. 6 shows results from the complex biological dataset dC1. (A)-(H): Distribution of the proteins associated with the respective pathways, which had been validated in the dataset by complementary transcriptome analysis; x axis is the protein order calculated by Normics' rank sum R, higher index correlates with higher DE likelihood according to our approach; (I): Cumulative distribution of the significant DE proteins identified by Normics$_{median}$; (J): Cumulative absolute fold changes (absolute log2 values summed up) of the significant DE proteins identified by Normics$_{median}$; (K): Number of significant DE proteins detected after normalization with the different algorithms; (L): Venn diagram of the significant DE proteins by five of the six algorithms (for better visualization); (M): Accordingly for the DE proteins belonging to either subset from subplots B-H.

In dCi, the higher Normics ranks (with high likelihood of being DE according to our approach) were enriched with the cross-validated DE pathways (and further DE proteins), while cytoskeleton-associated proteins (unlikely to be DE under the premises of the study) were primarily found with lower ranks (Fig. 6A-I). CYP4A12, a main DE finding of the original study, was placed in the 99th rank percentile by Normics, whereas GAPDH, a known housekeeping protein, was within the 5th rank percentile. The overall cumulative distribution of the fold changes of DE proteins was skewed to higher Normics ranks (Fig. 6J). Normics and Normics$_{median}$ identified the most and third most DE proteins respectively (Fig. 6K), even though normalization was based on a conservative 7 % (n=100) of all proteins only. Using Normics, unique DE proteins could be discovered, while the majority of identifications was shared with other algorithms (Fig. 6L). Identification of proteins from the cross-validated pathways was comparable across all algorithms (Fig. 6M).

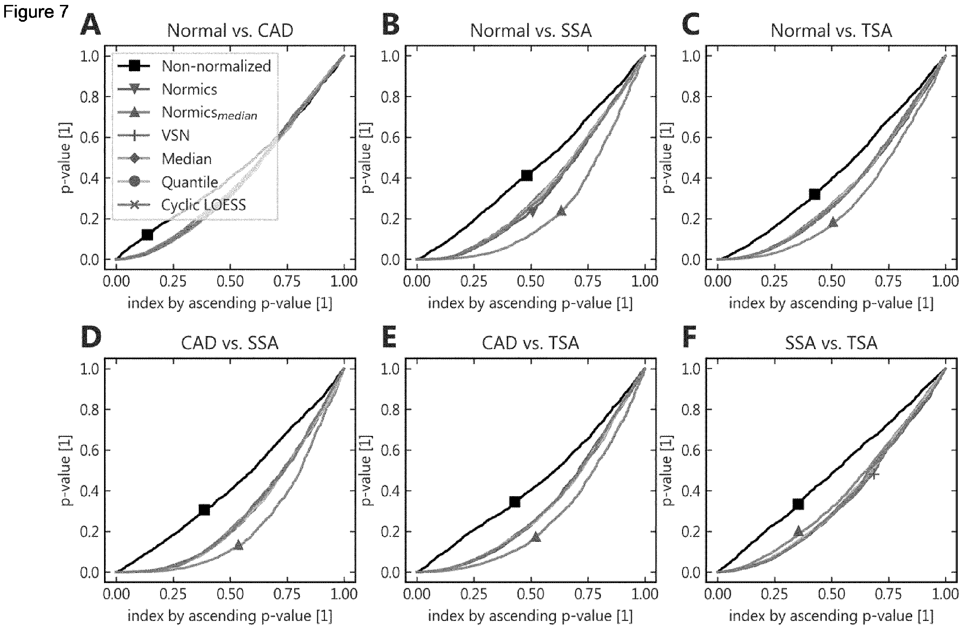

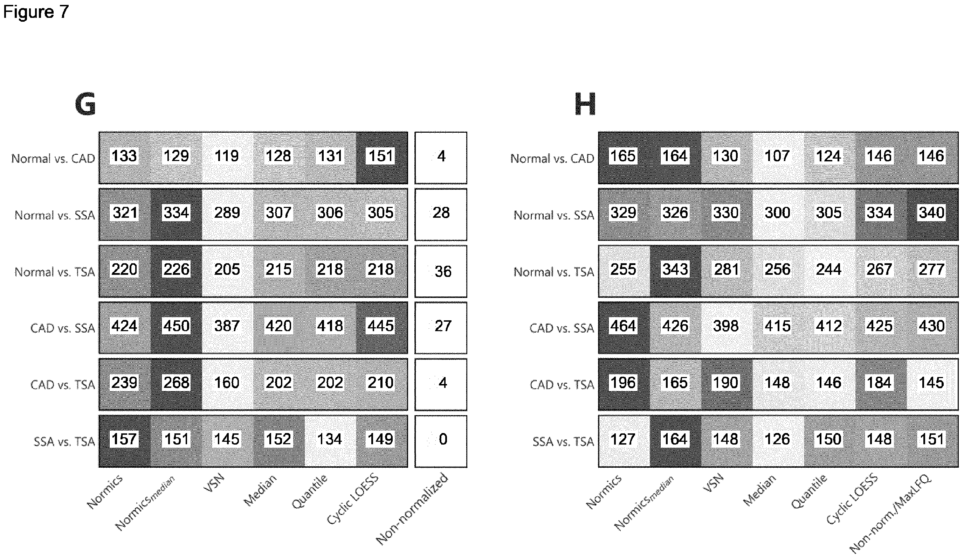

Fig. 7 shows results from complex pathological dataset dC2. (A)-(F): Quantile plot of the p-value distributions of the raw intensities normalized without fraction normalization (as included in MaxLFQ); (G): Heatmap summary of the number of proteins identified as DE (greyscale range per comparison, excluding the non-normalized input data); (H): In analogy to subplot G with MaxLFQ-normalized intensities as input; Normal := Normal mucosa; CAD := Conventional adenoma; SSA:= Sessile-serrated adenoma; TSA:= Traditional serrated adenoma.

dC2 offered more comparisons due to the variety of pathological subtypes covered by the dataset. N=250 (16 %) proteins were used for Normics normalization, more than in dC1 due to increased missingness in this dataset. In the raw-intensities dataset, the distribution of p-values (without FDR correction) was most markedly deviant from a uniform distribution for Normics$_{median}$ in 4 out of 6 comparisons (Fig. 7A-F). Both Normics variants increased identification of DE proteins markedly (Fig. 7G), even when the raw data was normalized with MaxLFQ first (Fig. 7H).

Fig. 8 shows Biological function of proteins ordered by Normics. The proteins of dataset dC2 were ordered by their Normics rank sum; The biological function of the 200 (12.5 %) proteins with the lowest and highest rank sums respectively were compared after functional annotation with Uniprot. The 200 proteins with the highest Normics ranks showed a broadened spectrum of biological functions compared to the subset with the lowest ranks.

EP 4 224 478 A1

## Discussion

Proteomic analyses in biomedical research are often used to evaluate complex cohorts with unknown structures. New patterns and biomarker candidates are sought and require high sensitivity and low numbers of false positives.

*ICS and CV-based identification of housekeeping proteins*

We tested our theoretical considerations to identify nDE housekeeping proteins based on their *CV* and mean correlation coefficient $\overline{\rho}$, our parameter for ICS. Simulated and spike-in data confirmed the stipulated relationship with circumscribed clusters of nDE and DE proteins. In complex datasets, with a lack of known true DE proteins, we found a similar data structure with an inverse correlation of *CV* and $\overline{\rho}$. We used a proteomic dataset from an animal study with cross-validation by transcriptome analysis. In this dataset, our algorithm attributed high likelihood of being DE to those proteins that in fact belonged to the regulated pathways.

Our algorithm does not depend on the *a priori* definition of sample groups, replicates, batches or other parameters (i.e. the experimental design matrix). This makes our approach more versatile and easier to use than e.g. mixed-effects models [32] and normalization extends beyond pre-defined sources of variation.

*Relative performance*

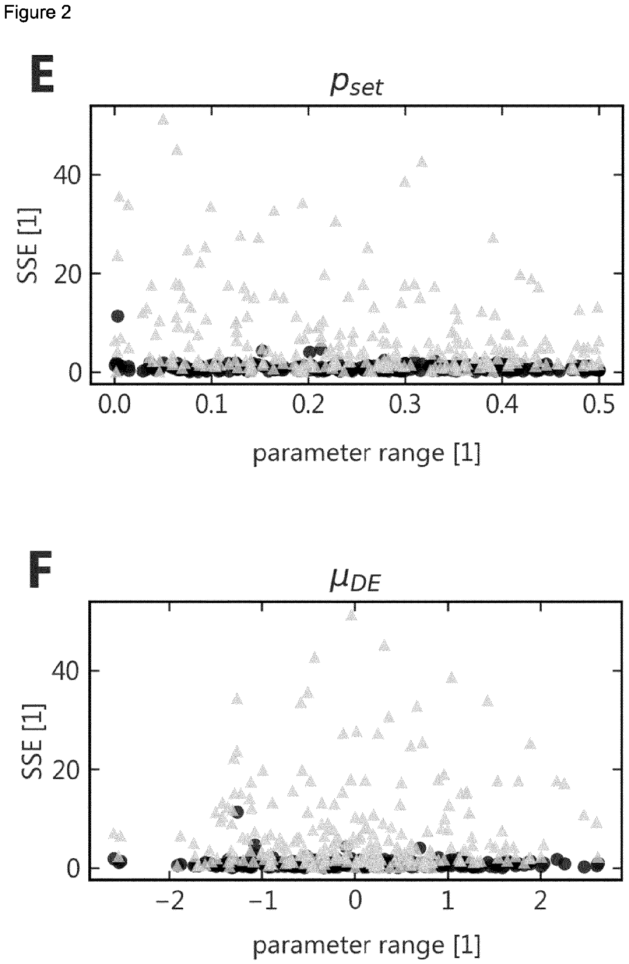

The similarity of protein expression patterns across samples was investigated in simulated proteomic data. Both Normics variants performed robustly and showed the lowest number of outliers compared to median, quantile, cyclic LOESS or VSN normalization. Normics with sequential VSN consistently outperformed VSN alone across the stochastically varied parameter space of the simulation (Fig. 2).

In spike-in datasets (Fig. 3-5) both Normics variants performed better than median, quantile, cyclic LOESS or VSN in the majority of cases. The stable performance with high sensitivity, low numbers of false positive DE proteins and neither over- nor underestimation of quantitative differences was unique to Normics and Normics$_{median}$. While the quantitative range in dS3 (UPS1 spike-in; [27]) was similarly retrieved by all algorithms, the share of DE proteins was very low in this dataset (3 %, Table 1), which limits its usability to discern algorithm performance.

Of note, VSN performed considerably worse in dS2 compared to all other datasets. Most likely this is due to a special feature of this dataset: The protein intensities of each sample were reported as ratios relative to a control sample. While this can be helpful, for instance when combining different fractions into a single quantitative readout, differences in average intensities were thus levelled out, blurring the variance-to-intensity relationship underlying VSN normalization. Although the share of DE proteins in this dataset was considerably lower than the chosen VSN quantile (0.2 vs. 0.5) sensitivity, specificity and quantitative data structure were markedly reduced. Normics, using VSN on the normalization subset only, did not suffer from such a distortion. Nonetheless Normics$_{median}$ should be used with data reported as ratios.

In complex datasets with relevant biological and pathological alterations (Fig. 6-7) both Normics variants were able to normalize the data based on only few proteins with higher numbers of DE proteins. Most of these identifications were shared with at least one other algorithm, indicating improved but not distorted normalization compared to the other algorithms. In 6 out of 7 comparisons of biological or pathological groups considerably more proteins were significantly identified as DE by the Normics approach. Cytoskeleton-related proteins and GAPDH, which are likely to be nDE (at least in the tissue of the same type) and are used as housekeeping controls [33, 34], were enriched in Normics normalization subsets in dC1. Members of cross-validated regulated pathways in dC1 were correctly excluded from the normalization subset by Normics. P-value distribution was most relevantly shifted to higher significance with Normics$_{median}$ in the complex pathological cohort dC2.

*Application for filtering of relevant DE proteins for downstream analysis*

Variance-based filtering of omics datasets has been proposed as a means to reduce the number of statistical tests, which can be helpful for reducing type II errors [20]. Normics creates a ranking list of all candidate proteins in the dataset that correlates with their likelihood of being DE. This list, which is provided separately by the algorithm, can be used to filter the data much in the same way. Normics' independence from *a priori* knowledge of experimental conditions ensures statistical independence for type I error control as outlined by Bourgon et al. [20]. In terms of the main use case of this work - explorative biomedical biomarker screens - this is particularly useful for unsupervised cluster analysis. Several of the most commonly used algorithms, such as non-negative matrix factorization [35], are iterative stochastic optimization problems. These can be prone to local minima and benefit from prefiltered input data [36].

12

*Combination with peptide level normalization*

Proteomic data can be normalized on both peptide and protein level. The combination of extracted ion currents from multiple fractions and samples into final peptide and protein quantities has been addressed by MaxLFQ [26]. This algorithm roots in minimizing the overall peptide variation and implies the assumption that a high share (undefined more than the majority) of proteins and peptides is nDE. As fractionation is usually not just a parallel replication of measurements but the application of an orthogonal method of peptide separation (resulting in bell-shaped peptide distributions across fractions), it is not clear whether this assumption always holds true. While MaxLFQ demonstrates robust performance in our comparison, downstream normalization with Normics reduces the number of false positives (dSi, Fig. 3G), corrects quantitative distortion (Fig. 3H) and increases the number of identified DE proteins (dC2, Fig. 7H). This hints at some variance being reduced by MaxLFQ (presumably on peptide/fraction level) while residuals remain. Care must be taken when unfractionated samples have been normalized with MaxLFQ: The main assumption of a majority of nDE proteins is then likely to introduce distortions that prevent normalization with Normics. Of note, Normics can be applied on peptide level too if there is reasonable evidence of "constitutively" prevalent peptides across fractions.

*Previous comparisons and proteome-specific approaches*

Several algorithms have been proposed specifically for proteomic data: DeqMS was developed by Zhu et al. [9] to reduce variance based on the number of peptides used for quantitation, building upon limma methods. MAP [10] was proposed as algorithm for the normalization of isobaric-labelled data with *a priori* defined experimental conditions, similar to an approach proposed by Zhang et al. [37]. These latter two were not investigated as we specifically set out to address the need for normalization methods independent of known biological groups. DeqMS focuses on the number peptide spectrum matches (PSM) as a prior to statistical DE testing and is, as such, not a normalization algorithm per se. It was therefore not included in the present comparison but can be combined with the additional information of PSM counts in downstream DE analysis.

In a systematic comparison Valinkangas et al. [7] investigated different normalization algorithms and found VSN to perform systematically well. In our analysis VSN demonstrated good normalization results but suffered from quantitative distortion in some cases (Fig. 4E), especially in relation to ratio-reported data (as explained above). In almost all cases including simulation data Normics could improve robustness and accuracy and identified more DE proteins. Apart from offering added value in providing a ranking list for downstream data filtering, Normics was the only algorithm that performed well in all scenarios. This underlines its usability in settings with unknown premises such as biomarker discovery screens across multiple conditions and stages.

Avoiding general assumptions about the share, extent and structure of true biological variance, we demonstrate for the first time the usability of the ICS for the normalization of omics data. We provide a theoretical link between mean correlation and nDE likelihood and embed our approach in both CV-based nDE subset selection and established normalization algorithms. The resulting Normics approach yields consistent results and outperforms standard algorithms in sensitivity, specificity and quantitative accuracy. The computation is straightforward and can be expanded to further types of omics data. *A priori* definition of an experiment design matrix is not necessary, and normalization is not limited to known factors of sample variation.

The measures for both variance and correlation structure can be varied to be based - for instance - on different correlation coefficients or different strategies of variance correction/estimation. For the former, in a preferred embodiment we chose Spearman's coefficient due to its relative robustness against heterogeneous data distributions (in which Pearson's coefficient may be susceptible to outliers). Further parameters and patterns such as the variances of the correlation coefficient may contain additional information. Iterative variants minimizing the mean correlation of the nDE subset are also possible.

**References**

1. Smyth, G.K., limma: Linear Models for Microarray Data, in Bioinformatics and Computational Biology Solutions Using R and Bioconductor. 2005. p. 397-420.

2. Chawade, A., E. Alexandersson, and F. Levander, Normalyzer: a tool for rapid evaluation of normalization methods for omics data sets. J Proteome Res, 2014. 13(6): p. 3114-20.

3. Pfaffl, M.W., et al., Determination of stable housekeeping genes, differentially regulated target genes and sample integrity: BestKeeper--Excel-based tool using pair-wise correlations. Biotechnol Lett, 2004. 26(6): p. 509-15.

4. Huber, W., et al., Variance stabilization applied to microarray data calibration and to the quantification of differential expression. Bioinformatics, 2002. 18 Suppl 1: p. S96-104.

5. Huber, W., et al., Parameter estimation for the calibration and variance stabilization of microarray data. Stat Appl Genet Mol Biol, 2003. 2: p. Article3.

6. Bolstad, B.M., et al., A comparison of normalization methods for high density oligonucleotide array data based on variance and bias. Bioinformatics, 2003. 19(2): p. 185-93.

7. Valikangas, T., T. Suomi, and L.L. Elo, A systematic evaluation of normalization methods in quantitative label-free proteomics. Brief Bioinform, 2018. 19(1): p. 1-11.

8. O'Rourke, M.B., et al., What is Normalization? The Strategies Employed in Top-Down and Bottom-Up Proteome Analysis Workflows. Proteomes, 2019. 7(3).

9. Zhu, Y., et al., DEqMS: A Method for Accurate Variance Estimation in Differential Protein Expression Analysis. Molecular & Cellular Proteomics, 2020. 19(6): p. 1047-1057.

10. Li, M., et al., MAP: model-based analysis of proteomic data to detect proteins with significant abundance changes. Cell Discov, 2019. 5: p. 40.

11. Walach, J., P. Filzmoser, and K. Hron, Data Normalization and Scaling: Consequences for the Analysis in Omics Sciences, in Data Analysis for Omic Sciences: Methods and Applications. 2018. p. 165-196.

12. Robinson, M.D. and A. Oshlack, A scaling normalization method for differential expression analysis of RNA-seq data. Genome Biol, 2010. 11(3): p. R25.

13. Evans, C., J. Hardin, and D.M. Stoebel, Selecting between-sample RNA-Seq normalization methods from the perspective of their assumptions. Brief Bioinform, 2018. 19(5): p. 776-792.

14. Risso, D., et al., Normalization of RNA-seq data using factor analysis of control genes or samples. Nat Biotechnol, 2014. 32(9): p. 896-902.

15. Washburn, M.P., D. Wolters, and J.R. Yates, 3rd, Large-scale analysis of the yeast proteome by multidimensional protein identification technology. Nat Biotechnol, 2001. 19(3): p. 242-7.

16. Liu, H., R.G. Sadygov, and J.R. Yates, 3rd, A model for random sampling and estimation of relative protein abundance in shotgun proteomics. Anal Chem, 2004. 76(14): p. 4193-201.

17. Pascovici, D., et al., Multiple testing corrections in quantitative proteomics: A useful but blunt tool. Proteomics, 2016. 16(18): p. 2448-53.

18. Duo, A., M.D. Robinson, and C. Soneson, A systematic performance evaluation of clustering methods for single-cell RNA-seq data. F1000Res, 2018. 7: p. 1141.

19. Love, M.I., W. Huber, and S. Anders, Moderated estimation offold change and dispersionfor RNA-seq data with DESeq2. Genome Biol, 2014. 15(12): p. 550.

20. Bourgon, R., R. Gentleman, and W. Huber, Independent filtering increases detection power for high-throughput experiments. Proc Natl Acad Sci USA, 2010. 107(21): p. 9546-51.

21. Kohsler, M., et al., Validation of reference genes for the normalization of RT-qPCR gene expression in Acanthamoeba spp. Sci Rep, 2020. 10(1): p. 10362.

22. Sarwar, M.B., et al., Identification and validation of superior housekeeping gene(s) for qRT-PCR data normalization in Agave sisalana (a CAM-plant) under abiotic stresses. Physiol Mol Biol Plants, 2020. 26(3): p. 567-584.

23. Czechowski, T., et al., Genome-wide identification and testing of superior reference genes for transcript normalization in Arabidopsis. Plant Physiol, 2005. 139(1): p. 5-17.

24. Calza, S., D. Valentini, and Y. Pawitan, Normalization of oligonucleotide arrays based on the least-variant set of genes. BMC Bioinformatics, 2008. 9: p. 140.

25. Suo, C., et al., Modified least-variant set normalization for miRNA microarray. RNA, 2010. 16(12): p. 2293-303.

26. Cox, J., et al., Accurate proteome-wide label-free quantification by delayed normalization and maximal peptide ratio extraction, termed MaxLFQ. Mol Cell Proteomics, 2014. 13(9): p. 2513-26.

27. Pursiheimo, A., et al., Optimization of Statistical Methods Impact on Quantitative Proteomics Data. J Proteome Res, 2015. 14(10): p. 4118-26.

28. Vehmas, A.P., et al., Liver lipid metabolism is altered by increased circulating estrogen to androgen ratio in male mouse. J Proteomics, 2016. 133:p. 66-75.

29. Sohier, P., et al., Proteome analysis offormalin-fixed paraffin-embedded colorectal adenomas reveals the heterogeneous nature of traditional serrated adenomas compared to other colorectal adenomas. J Pathol, 2020. 250(3): p. 251-261.

30. Seabold, S. and J. Perktold, Statsmodels: Econometric and Statistical Modeling with Python, in Proceedings of the 9th Python in Science Conference. 2010. p. 92-96.

31. Heberle, H., et al., InteractiVenn: a web-based tool for the analysis of sets through Venn diagrams. BMC Bioinformatics, 2015. 16: p. 169.

32. Hoffman, G.E. and E.E. Schadt, variancePartition: interpreting drivers of variation in complex gene expression studies. BMC Bioinformatics, 2016. 17(1): p. 483.

33. Sun, S., et al., A protein-based set of reference markers for liver tissues and hepato-cellular carcinoma. BMC Cancer, 2009. 9(1).

34. Barber, R.D., et al., GAPDH as a housekeeping gene: analysis of GAPDH mRNA expression in a panel of 72 human tissues. Physiol Genomics, 2005. 21(3): p. 389-95.

35. Kim, H. and H. Park, Sparse non-negative matrix factorizations via alternating non-negativity-constrained least squares for microarray data analysis. Bioinformatics, 2007. 23(12): p. 1495-502.

36. Tritchler, D., E. Parkhomenko, and J. Beyene, Filtering genes for cluster and network analysis. BMC Bioinformatics, 2009. 10: p. 193.

37. Zhang, Y., et al., A robust error model for iTRAQ quantification reveals divergent signaling between oncogenic FLT3 mutants in acute myeloid leukemia. Mol Cell Proteomics, 2010. 9(5): p. 780-90.

38. Piehowski, P.D., et al., Sources of technical variability in quantitative LC-MS proteomics: human brain tissue sample analysis. J Proteome Res, 2013. 12(5): p. 2128-37.

39. Wu, D., et al., Deciphering global signal features of high-throughput array data from cancers. Mol Biosyst, 2014. 10(6): p. 1549-56.

40. Rousseeuw, P.J. and A.M. Leroy, Robust Regression and Outlier Detection. Wiley Series in Probability and Statistics. 1987.

## Claims

1. A method for determining differential expression of a test analyte, the method comprising:

   - obtaining quantitative measurements corresponding to a plurality of analytes, including the test analyte,
   - determining a variation of the test analyte across the measurements of the test analyte and/or a correlation of the measurements of the test analyte with measurements of other analytes from the plurality of analytes, and
   - determining a differentially expressed, DE, or non-differentially-expressed, nDE, likelihood of the test analyte based on the variation and/or the correlation.

2. The method of claim 1, wherein the analyte is a gene, a metabolite, a lipid, an ion channel, pathogens, cells or cell types.

3. The method of claim 1 or 2, wherein the determining the variation comprises determining a coefficient of variation, in particular a coefficient of variation that is given by

$$\frac{\sqrt{Var(g_i)}}{\bar{g}_i}$$

wherein $Var(g_i)$ is a variation of measurements of the test analyte and $\bar{g}_i$ is a mean of an abundance $g_i$ of measurements of the test analyte.

4. The method of one of the previous claims, further comprising a step of correcting a value of the variance of the test analyte based on a number of peptides used for quantification and/or based on a number of read variants.

5. The method of one of the previous claims, wherein the determining the correlation comprises determining a correlation coefficient, in particular a mean correlation coefficient, preferably the mean correlation coefficient $\overline{\rho}(g_i)$ that is determined by:

$$\bar{\rho}(g_i) = \frac{1}{N-1} \sum_{n=1}^{N \backslash i} \rho(g_i, g_n)$$

wherein $g_i$ is the test analyte, $N$ is a number of the plurality of analytes, $\rho$ is a correlation coefficient, preferably a Spearman correlation coefficient.

6. The method of one of the previous claims, wherein the determining the DE or nDE likelihood of the test analyte comprises ranking the plurality of analytes based on the variation and/or the correlation.

7. The method of claim 6, wherein the ranking $R_i$ of the test analyte is determined as a sum of a first ranking function that assigns a higher ranking to a higher variation and a second ranking function that assigns a higher ranking to a lower mean correlation.

8. The method of claims 6 or 7, wherein the ranking of the analytes by DE likelihood is used to filter the data for analytes with high DE likelihood in order to improve sensitivity and performance of downstream statistical analyses, such as unsupervised clustering, principal component analysis or statistical testing for differential expression.

9. The method of one of the previous claims, wherein the method is a normalization method that comprises a further step of normalizing measurements based on one or more analytes with nDE status.

10. The method of one of the previous claims, wherein the method comprises computing a symmetric correlation matrix and computing a mean of each column or row of the symmetric correlation matrix.

11. The method of one of the previous claims, wherein the method comprises a further step of determining a subset of the plurality of analytes and the determining a correlation of the measurements of the test analyte with measurements of other analytes from the plurality of analytes comprises determining the correlation of the test analyte only with other analytes that are part of the subset.

12. The method of claim 11, wherein analytes of the subset are determined randomly and/or based on an abundance of the analytes.

13. An apparatus for determining differential expression of a test analyte, wherein the apparatus is configured to carry out the method of one of the previous claims.

14. The apparatus of claim 13, wherein the apparatus further comprises a mass spectrometer and wherein the apparatus is configured to configured to access spectral libraries via a network connection.

15. A computer-readable storage medium storing program code, the program code comprising instructions that when executed by a processor carry out the method of one of claims 1 to 12.

Figure 1

**A** Simulation

**B** Spike-in

Figure 1

Figure 1

Figure 2

Figure 2

Figure 2

E $p_{set}$

F $\mu_{DE}$

Figure 2

**G** $\sigma_{DE}$

**H** $Q_{DE}$

Figure 3

Figure 3

Figure 4

Figure 4

Figure 5

**A** Sensitivity

| | Normics | Normics median | VSN | Median | Quantile | Cyclic LOESS |
|---|---|---|---|---|---|---|
| 2 vs. 4 fmol | 0.0 | 0.0 | 0.0 | 0.0 | 0.0 | 0.0 |
| 2 vs. 10 fmol | 91.49 | 91.49 | 87.23 | 91.49 | 95.74 | 87.23 |
| 2 vs. 25 fmol | 100.0 | 100.0 | 100.0 | 100.0 | 97.87 | 100.0 |
| 2 vs. 50 fmol | 100.0 | 100.0 | 100.0 | 100.0 | 100.0 | 100.0 |
| 4 vs. 10 fmol | 4.26 | 19.15 | 2.13 | 2.13 | 4.26 | 2.13 |
| 4 vs. 25 fmol | 97.87 | 100.0 | 100.0 | 97.87 | 97.87 | 97.87 |
| 4 vs. 50 fmol | 100.0 | 100.0 | 100.0 | 100.0 | 97.87 | 100.0 |
| 10 vs. 25 fmol | 63.83 | 59.57 | 78.72 | 61.7 | 65.96 | 70.21 |
| 10 vs. 50 fmol | 91.49 | 91.49 | 91.49 | 91.49 | 89.36 | 91.49 |
| 25 vs. 50 fmol | 0.0 | 0.0 | 0.0 | 0.0 | 0.0 | 0.0 |

**B** Specificity

| | Normics | Normics median | VSN | Median | Quantile | Cyclic LOESS |
|---|---|---|---|---|---|---|
| 2 vs. 4 fmol | 100.0 | 100.0 | 100.0 | 100.0 | 99.93 | 100.0 | 100.0 |
| 2 vs. 10 fmol | 86.37 | 89.5 | 92.78 | 88.7 | 85.35 | 93.37 |
| 2 vs. 25 fmol | 47.96 | 53.64 | 57.36 | 56.78 | 48.03 | 59.62 |
| 2 vs. 50 fmol | 38.05 | 40.67 | 46.28 | 36.95 | 36.3 | 46.21 |
| 4 vs. 10 fmol | 90.67 | 91.91 | 95.48 | 92.93 | 96.87 | 96.94 |
| 4 vs. 25 fmol | 51.24 | 58.31 | 61.01 | 62.76 | 52.11 | 61.22 |
| 4 vs. 50 fmol | 38.56 | 43.73 | 47.96 | 41.55 | 39.8 | 49.64 |
| 10 vs. 25 fmol | 98.1 | 98.25 | 97.3 | 98.4 | 94.39 | 97.89 |
| 10 vs. 50 fmol | 89.21 | 89.94 | 87.76 | 82.29 | 80.17 | 89.29 |
| 25 vs. 50 fmol | 99.78 | 99.78 | 100.0 | 99.93 | 99.78 | 99.78 |

Figure 5

Figure 6

**A** Cytoskeleton organization (nDE)

**B** Fatty acid metabolism (DE)

**C** Terpenoid (DE)

**D** Steroid metabolism (DE)

Figure 6

E Complement activation (DE)

F Lipid hydroxylation (DE)

G Xenobiotic metabolism (DE)

H Peroxisome (DE)

Figure 6

Figure 6

**L**

Normics
(348)

Cyclic Loess
(324)

VSN
(309)

19

1        17
4   5   0   7
9      6      2   1      9
15         0
2      230      3   1
0  16         0
5      4   19   2   4
2   1
1

7                4

Quantile
(300)

Median
(279)

**M**

Normics
(32)

Cyclic Loess
(34)

VSN
(31)

1

0        0
1   0   0   1
1      0      0   0      0
0         0
0      26      1   1
0  2         0
0      0   1   1
0   0
0

1                0

Quantile
(33)

Median
(31)

Figure 7

EP 4 224 478 A1

Figure 7

**G**

| | Normics | Normics:median | VSN | Median | Quantile | Cyclic LOESS | Non-normalized |
|---|---|---|---|---|---|---|---|
| Normal vs. CAD | 133 | 129 | 119 | 128 | 131 | 151 | 4 |
| Normal vs. SSA | 321 | 334 | 289 | 307 | 306 | 305 | 28 |
| Normal vs. TSA | 220 | 226 | 205 | 215 | 218 | 218 | 36 |
| CAD vs. SSA | 424 | 450 | 387 | 420 | 418 | 445 | 27 |
| CAD vs. TSA | 239 | 268 | 160 | 202 | 202 | 210 | 4 |
| SSA vs. TSA | 157 | 151 | 145 | 152 | 134 | 149 | 0 |

**H**

| | Normics | Normics:median | VSN | Median | Quantile | Cyclic LOESS | Non-norm./MaxLFQ |
|---|---|---|---|---|---|---|---|
| Normal vs. CAD | 165 | 164 | 130 | 107 | 124 | 146 | 146 |
| Normal vs. SSA | 329 | 326 | 330 | 300 | 305 | 334 | 340 |
| Normal vs. TSA | 255 | 343 | 281 | 256 | 244 | 267 | 277 |
| CAD vs. SSA | 464 | 426 | 398 | 415 | 412 | 425 | 430 |
| CAD vs. TSA | 196 | 165 | 190 | 148 | 146 | 184 | 145 |
| SSA vs. TSA | 127 | 164 | 148 | 126 | 150 | 148 | 151 |

35

Figure 8

EP 4 224 478 A1

Figure 9

NORMICS

Select input data file

| | |
|---|---|
| Minimum share of samples per protein | 0.8 |
| Reduce correlation by [1 = No reduction] | 1 |
| # of cores not to use for computation | 1 |
| Re-run with existing computation | ☐ |

_____ Additional settings _____

| | |
|---|---|
| Handle zero intensities as None | ☑ |
| Reverse log2 | ☐ |
| Reverse log | ☐ |
| Reduction of input data [1 = No reduction] | 1 |
| VSN_quantile | 0.5 |
| VSN_quantile for Normics subset | 0.8 |

Start

Cancel

Europäisches
Patentamt

European
Patent Office

Office européen
des brevets

# EUROPEAN SEARCH REPORT

Application Number

EP 22 15 5644

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YANG YIN ET AL: "StatsPro: Systematic integration and evaluation of statistical approaches for detecting differential expression in label-free quantitative proteomics", JOURNAL OF PROTEOMICS, vol. 250, 30 September 2021 (2021-09-30), page 104386, XP055939407, AMSTERDAM, NL ISSN: 1874-3919, DOI: 10.1016/j.jprot.2021.104386 * the whole document * ----- | 1-15 | INV. G16B25/10 |
| X | VÄLIKANGAS TOMMI ET AL: "A systematic evaluation of normalization methods in quantitative label-free proteomics", BRIEFINGS IN BIOINFORMATICS, 2 October 2016 (2016-10-02), page bbw095, XP055939677, GB ISSN: 1467-5463, DOI: 10.1093/bib/bbw095 * the whole document * ----- | 1-15 | |
| | | | **TECHNICAL FIELDS SEARCHED (IPC)** |
| | | | G16B |

The present search report has been drawn up for all claims

| Place of search | Date of completion of the search | Examiner |
|---|---|---|
| Munich | 7 July 2022 | Díaz de Lezana, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SMYTH, G.K.** limma: Linear Models for Microarray Data. *Bioinformatics and Computational Biology Solutions Using R and Bioconductor,* 2005, 397-420
- **CHAWADE, A. ; E. ALEXANDERSSON ; F. LEVANDER.** Normalyzer: a tool for rapid evaluation of normalization methods for omics data sets. *J Proteome Res,* 2014, vol. 13 (6), 3114-20
- **PFAFFL, M.W. et al.** Determination of stable housekeeping genes, differentially regulated target genes and sample integrity: BestKeeper--Excel-based tool using pair-wise correlations. *Biotechnol Lett,* 2004, vol. 26 (6), 509-15
- **HUBER, W. et al.** Variance stabilization applied to microarray data calibration and to the quantification of differential expression. *Bioinformatics,* 2002, vol. 18 (1), 96-104
- **HUBER, W. et al.** Parameter estimation for the calibration and variance stabilization of microarray data. *Stat Appl Genet Mol Biol,* 2003, vol. 2
- **BOLSTAD, B.M. et al.** A comparison of normalization methods for high density oligonucleotide array data based on variance and bias. *Bioinformatics,* 2003, vol. 19 (2), 185-93
- **VALIKANGAS, T. ; T. SUOMI ; L.L. ELO.** A systematic evaluation of normalization methods in quantitative label-free proteomics. *Brief Bioinform,* 2018, vol. 19 (1), 1-11
- **O'ROURKE, M.B. et al.** What is Normalization? The Strategies Employed in Top-Down and Bottom-Up Proteome Analysis Workflows. *Proteomes,* 2019, vol. 7 (3
- **ZHU, Y. et al.** DEqMS: A Method for Accurate Variance Estimation in Differential Protein Expression Analysis. *Molecular & Cellular Proteomics,* 2020, vol. 19 (6), 1047-1057
- **LI, M. et al.** MAP: model-based analysis of proteomic data to detect proteins with significant abundance changes. *Cell Discov,* 2019, vol. 5, 40
- **WALACH, J. ; P. FILZMOSER ; K. HRON.** Data Normalization and Scaling: Consequences for the Analysis in Omics Sciences. *Data Analysis for Omic Sciences: Methods and Applications.,* 2018, 165-196
- **ROBINSON, M.D. ; A. OSHLACK.** A scaling normalization method for differential expression analysis of RNA-seq data. *Genome Biol,* 2010, vol. 11 (3), R25
- **EVANS, C. ; J. HARDIN ; D.M. STOEBEL.** Selecting between-sample RNA-Seq normalization methods from the perspective of their assumptions. *Brief Bioinform,* 2018, vol. 19 (5), 776-792
- **RISSO, D. et al.** Normalization of RNA-seq data using factor analysis of control genes or samples. *Nat Biotechnol,* 2014, vol. 32 (9), 896-902
- **WASHBURN, M.P. ; D. WOLTERS ; J.R. YATES, 3.** Large-scale analysis of the yeast proteome by multidimensional protein identification technology. *Nat Biotechnol,* 2001, vol. 19 (3), 242-7
- **LIU, H. ; R.G. SADYGOV ; J.R. YATES, 3.** A model for random sampling and estimation of relative protein abundance in shotgun proteomics. *Anal Chem,* 2004, vol. 76 (14), 4193-201
- **PASCOVICI, D. et al.** Multiple testing corrections in quantitative proteomics: A useful but blunt tool. *Proteomics,* 2016, vol. 16 (18), 2448-53
- **DUO, A. ; M.D. ROBINSON ; C. SONESON.** A systematic performance evaluation of clustering methods for single-cell RNA-seq data. *F1000Res,* 2018, vol. 7, 1141
- **LOVE, M.I. ; W. HUBER ; S. ANDERS.** Moderated estimation offold change and dispersionfor RNA-seq data with DESeq2. *Genome Biol,* 2014, vol. 15 (12), 550
- **BOURGON, R. ; R. GENTLEMAN ; W. HUBER.** Independent filtering increases detection power for high-throughput experiments. *Proc Natl Acad Sci USA,* 2010, vol. 107 (21), 9546-51
- **KOHSLER, M. et al.** Validation of reference genes for the normalization of RT-qPCR gene expression in Acanthamoeba spp. *Sci Rep,* 2020, vol. 10 (1), 10362
- **SARWAR, M.B. et al.** Identification and validation of superior housekeeping gene(s) for qRT-PCR data normalization in Agave sisalana (a CAM-plant) under abiotic stresses. *Physiol Mol Biol Plants,* 2020, vol. 26 (3), 567-584
- **CZECHOWSKI, T. et al.** Genome-wide identification and testing of superior reference genes for transcript normalization in Arabidopsis. *Plant Physiol,* 2005, vol. 139 (1), 5-17
- **CALZA, S. ; D. VALENTINI ; Y. PAWITAN.** Normalization of oligonucleotide arrays based on the least-variant set of genes. *BMC Bioinformatics,* 2008, vol. 9, 140

- **SUO, C. et al.** Modified least-variant set normalization for miRNA microarray. *RNA,* 2010, vol. 16 (12), 2293-303
- **COX, J. et al.** Accurate proteome-wide label-free quantification by delayed normalization and maximal peptide ratio extraction, termed MaxLFQ. *Mol Cell Proteomics,* 2014, vol. 13 (9), 2513-26
- **PURSIHEIMO, A. et al.** Optimization of Statistical Methods Impact on Quantitative Proteomics Data. *J Proteome Res,* 2015, vol. 14 (10), 4118-26
- **VEHMAS, A.P. et al.** Liver lipid metabolism is altered by increased circulating estrogen to androgen ratio in male mouse. *J Proteomics,* 2016, vol. 133, 66-75
- **SOHIER, P. et al.** Proteome analysis offormalin-fixed paraffin-embedded colorectal adenomas reveals the heterogeneous nature of traditional serrated adenomas compared to other colorectal adenomas. *J Pathol,* 2020, vol. 250 (3), 251-261
- **SEABOLD, S ; J. PERKTOLD.** Statsmodels: Econometric and Statistical Modeling with Python. *Proceedings of the 9th Python in Science Conference,* 2010, 92-96
- **HEBERLE, H. et al.** InteractiVenn: a web-based tool for the analysis of sets through Venn diagrams. *BMC Bioinformatics,* 2015, vol. 16, 169
- **HOFFMAN, G.E. ; E.E. SCHADT.** variancePartition: interpreting drivers of variation in complex gene expression studies. *BMC Bioinformatics,* 2016, vol. 17 (1), 483
- **SUN, S. et al.** A protein-based set of reference markers for liver tissues and hepato-cellular carcinoma. *BMC Cancer,* 2009, vol. 9 (1
- **BARBER, R.D. et al.** GAPDH as a housekeeping gene: analysis of GAPDH mRNA expression in a panel of 72 human tissues. *Physiol Genomics,* 2005, vol. 21 (3), 389-95
- **KIM, H. ; H. PARK.** Sparse non-negative matrix factorizations via alternating non-negativity-constrained least squares for microarray data analysis. *Bioinformatics,* 2007, vol. 23 (12), 1495-502
- **TRITCHLER, D. ; E. PARKHOMENKO ; J. BEYENE.** Filtering genes for cluster and network analysis. *BMC Bioinformatics,* 2009, vol. 10, 193
- **ZHANG, Y. et al.** A robust error model for iTRAQ quantification reveals divergent signaling between oncogenic FLT3 mutants in acute myeloid leukemia. *Mol Cell Proteomics,* 2010, vol. 9 (5), 780-90
- **PIEHOWSKI, P.D. et al.** Sources of technical variability in quantitative LC-MS proteomics: human brain tissue sample analysis. *J Proteome Res,* 2013, vol. 12 (5), 2128-37
- **WU, D. et al.** Deciphering global signal features of high-throughput array data from cancers. *Mol Biosyst,* 2014, vol. 10 (6), 1549-56
- **ROUSSEEUW, P.J. ; A.M. LEROY.** Robust Regression and Outlier Detection. *Wiley Series in Probability and Statistics.,* 1987